# EUROPÄISCHE PATENTSCHRIFT

(11) **EP 1 578 416 B1**
(45) Veröffentlichungstag und Bekanntmachung des Hinweises auf die Patenterteilung: **02.08.2006**
(21) Anmeldenummer: 03795918.6
(22) Anmeldetag: 18.12.2003
(51) Int. Cl.: A61K 31/40, A61K 31/435, A61P 25/24, A61P 25/28, A61P 29/00

(54) **ARZNEIMITTEL ENTHALTEND SUBSTITUIERTE 2,5-DIAMINOMETHYL-1H-PYRROLE**
MEDICAMENTS CONTAINING SUBSTITUTED 2,5-DIAMINOMETHYL-1H-PYRROLES
MEDICAMENTS CONTENANT DES 2,5-DIAMINOMETHYL-1H-PYRROLES SUBSTITUES

(30) Priorität: 20.12.2002 DE 10261130
(43) Veröffentlichungstag der Anmeldung: 28.09.2005
(73) Patentinhaber: Grünenthal GmbH, 52078 Aachen (DE)
(72) Erfinder: MERLA, Beatrix, 52078 Aachen (DE); SUNDERMANN, Corinna, 52066 Aachen (DE); JAGUSCH, Utz-Peter, 52066 Aachen (DE); ENGLBERGER, Werner, 52223 Stolberg (DE); HENNIES, Hagen-Heinrich, 52152 Simmerath (DE)
(74) Vertreter: Brosch, Oliver
(86) Internationale Anmeldenummer: PCT/EP2003/014495
(87) Internationale Veröffentlichungsnummer: WO 2004/058248

(56) Entgegenhaltungen:
- EP-A- 0 312 345
- CHAKRABORTY T K ET AL: "Development of 5-(aminomethyl)pyrrole-2-carboxylic acid as a constrained surrogate of Gly-Alanine and its application in peptidomimetic studies" TETRAHEDRON LETTERS, ELSEVIER SCIENCE PUBLISHERS, AMSTERDAM, NL, Bd. 43, 2002, Seiten 2589-2592, XP002276009 ISSN: 0040-4039
- BACHMAN: "the condensation of aldheydes and amines..." JOURNAL OF THE AMERICAN CHEMICAL SOCIETY (1946), 68, 2496-9, 1946, Seiten 2496-9, XP001182202
- HUANG, JUI-HSIEN; KUO, PEI-CHENG; LEE, GENE-HSIANG; PENG: "Synthesis and structure characterization of ..." JOURNAL OF THE CHINESE CHEMICAL SOCIETY (TAIPEI) (2000), 47(6), 1191-1195, 2000, Seiten 1191-1195, XP001182204
- SCOTT M K ET AL: "PIPERAZINYLALKYL HETEROCYCLES AS POTENTIAL ANTIPSYCHOTIC AGENTS" JOURNAL OF MEDICINAL CHEMISTRY, AMERICAN CHEMICAL SOCIETY. WASHINGTON, US, Bd. 38, Nr. 21, 1995, Seiten 4198-4210, XP002092071 ISSN: 0022-2623
- CALO GUERRINI RIZZI: "Pharmacology of nociceptin and its receptor: a novel therapeutic target" BRITISH JOURNAL OF PHARMACOLOGY, Bd. 129, 2000, Seiten 1261-1283,

## Beschreibung

Die vorliegende Erfindung betrifft Arzneimittel enthaltend substituierte 2,5-Diaminomethyl-1 H-pyrrole sowie die Verwendung dieser Verbindungen zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide, wie beispielsweise das Morphin, sind bei der Therapie starker bis sehr starker Schmerzen wirksam, weisen jedoch unerwünschte Begleiterscheinungen, wie beispielsweise Atemdepression, Erbrechen, Sedierung oder Obstipation, auf. Nach weiteren schmerzhemmenden Mitteln wird weltweit geforscht.

Aufgabe der vorliegenden Erfindung war es daher, neue Arzneimittel zur Verfügung zu stellen, die sich insbesondere zur Bekämpfung von Schmerzen eignen.

Diese Aufgabe wurde durch die erfindungsgemäßen Arzneimittel enthaltend wenigstens ein substituiertes 2,5-Diaminomethyl-1 H-pyrrol der nachstehenden allgemeinen Formel I gelöst. Dokument EP 312345 offenbart die Aktivität von 2,5-Diaminomethyl-1H-pyrrole als Antipsychotika.

Es wurde überraschenderweise gefunden, daß die substituierten 2,5-Diaminomethyl-1 H-pyrrole der nachstehenden allgemeinen Formel I eine hohe Affinität für den ORL("opioid receptor like")-1-Rezeptor wie auch für den µ-Opioid-Rezeptor aufweisen und sich daher zur Regulation dieser Rezeptoren eignen.

Femer führen die substituierten 2,5-Diaminomethyl-1 H-pyrrole der nachstehenden allgemeinen Formel 1 zur Inhibierung des Noradrenalin-Uptakes sowie zur Inhibierung des 5-Hydroxytryptamin-(5-HT)-Uptakes.

Die erfindungsgemäßen Arzneimittel enthaltend wenigstens ein substituiertes 2,5-Diaminomethyl-1 H-pyrrol der nachstehenden allgemeinen Formel I zeigen insbesondere eine ausgeprägte Wirksamkeit bei der Bekämpfung von Schmerzen, vorzugsweise chronischen Schmerzen und/oder akuten Schmerzen und/oder neuropathischen Schmerzen.

Des weiteren eignen sich die erfindungsgemäßen Arzneimittel auch zur Behandlung von Entzugserscheinungen, Gedächtnis-Störungen, neurodegenerativen Erkrankungen, vorzugsweise Morbus Parkinson, Morbus Huntington oder Morbus Alzheimer, Epilepsie, Störungen des cardiovaskulären Systems, Wasserspeicher-Krankheiten, intestinaler Motilität (Diarrhoe), Harninkontinenz, Anorexie, Pruritus, Depressionen, Tinnitus, sexueller Dysfunktionen, vorzugsweise erektiler Dysfunktionen, Atemwegserkrankungen, oder zur Diurese, zur Beeinflussung des cardiovaskulären Systems, vorzugsweise zur Vasodilatation der Arterien, zur Unterdrückung des Miktionsreflexes, zur Anxiolyse, zur Regulation des Elektrolyt-Haushaltes, zur Regulation, vorzugsweise Stimulation, der Nahrungsaufnahme, zur Reduzierung des Suchtpotentials von Opioiden, insbesondere von Morphin, zur Modulation der Bewegungsaktivität, zur Beeinflussung der Wirkung von µ-Agonisten, insbesondere Morphin.

Ein Gegenstand der vorliegenden Erfindung ist daher ein Arzneimittel enthaltend ein oder mehrere substituierte 2,5-Diaminomethyl-1 H-pyrrole der nachstehenden allgemeinen Formel I 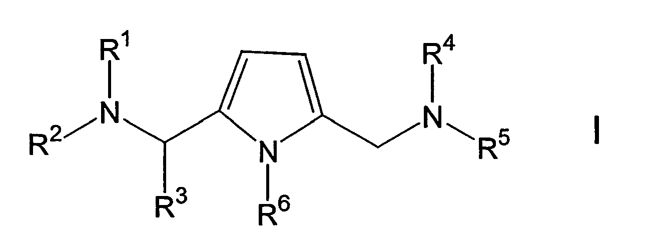 worin

R¹ für Wasserstoff, einen linearen oder verzweigten, gesättigten oder ungesättigten, ggf. wenigstens einfach substituierten aliphatischen Rest, einen ggf. über eine ggf. wenigstens einfach substituierte Alkylen-Gruppe gebundenen, gesättigten oder ungesättigten, ggf. wenigstens einfach substituierten, ggf. wenigstens ein Heteroatom als Ringglied aufweisenden cycloaliphatischen Rest, einen ggf. wenigstens einfach substituierten Aryl- oder Heteroaryl-Rest oder für einen über eine ggf. substituierte Alkylen-Gruppe gebundenen, ggf. wenigstens einfach substituierten Aryl- oder Heteroarylrest steht,

R² für einen linearen oder verzweigten, gesättigten oder ungesättigten, ggf. wenigstens einfach substituierten aliphatischen Rest, einen ggf. über eine ggf. wenigstens einfach substituierte Alkylen-Gruppe gebundenen, gesättigten oder ungesättigten, ggf. wenigstens einfach substituierten, ggf. wenigstens ein Heteroatom als Ringglied aufweisenden cycloaliphatischen Rest, einen ggf. wenigstens einfach substituierten Aryl- oder Heteroaryl-Rest oder für einen über eine ggf. substituierte Alkylen-Gruppe gebundenen, ggf. wenigstens einfach substituierten Aryl- oder Heteroarylrest, steht, oder

R¹ und R² gemeinsam mit dem sie verbindenden Stickstoffatom als Ringglied einen gesättigten oder ungesättigten, ggf. wenigstens ein weiteres Heteroatom als Ringglied aufweisenden, ggf. wenigstens einfach substituierten cycloaliphatischen Rest bilden,

R³ für einen ggf. wenigstens einfach substituierten Aryl- oder Heteroarylrest, eine Estergruppe oder eine Carboxygruppe steht,

R⁴ und R⁵, gleich oder verschieden, für Wasserstoff, einen linearen oder verzweigten, gesättigten oder ungesättigten, ggf. wenigstens einfach substituierten aliphatischen Rest, einen ggf. über eine ggf. wenigstens einfach substituierte Alkylen-Gruppe gebundenen, gesättigten oder ungesättigten, ggf. wenigstens einfach substituierten, ggf. wenigstens ein Heteroatom als Ringglied aufweisenden cycloaliphatischen Rest, einen ggf. wenigstens einfach substituierten Aryl- oder Heteroaryl-Rest, einen über eine ggf. wenigstens einfach substiuierte Alkylen-Gruppe gebundenden, ggf. wenigstens einfach substituierten Aryl- oder Heteroarylrest stehen,
oder

R⁴ und R⁵ gemeinsam mit dem sie verbindenden Stickstoffatom als Ringglied einen gesättigten oder ungesättigten, ggf. wenigstens ein weiteres Heteroatom als Ringglied aufweisenden, ggf. wenigstens einfach substituierten cycloaliphatischen Rest bilden, und

R⁶ für einen linearen oder verzweigten, gesättigten oder ungesättigten, ggf. wenigstens einfach substituierten aliphatischen Rest, einen ggf. über eine ggf. wenigstens einfach substituierte Alkylen-Gruppe gebundenen, gesättigten oder ungesättigten, ggf. wenigstens einfach substituierten, ggf. wenigstens ein Heteroatom als Ringglied aufweisenden cycloaliphatischen Rest, einen ggf. wenigstens einfach substituierten Aryl- oder Heteroaryl-Rest oder einen über eine ggf. wenigstens einfach substiuierte Alkylen-Gruppe geburidenden, ggf. wenigstens einfach substituierten Aryl- oder Heteroarylrest steht,

gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

Vorzugsweise kommen solche 2,5-Diaminomethyl-1 H-pyrrole der allgemeinen Formel für die erfindungsgemäßen Arzneimittel in Betracht, in denen R¹ für Wasserstoff, einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, fünf- oder sechsgliedrigen Aryl- oder Heteroarylrest oder einen über eine ggf. substituierte C₁₋₃-Alkylen-Gruppe gebundenen, ggf. wenigstens einfach substituierten, fünf- oder sechsgliedrigen Aryl- oder Heteroarylrest, vorzugsweise für einen linearen oder verzweigten, gesättigten aliphatischen C₁₋₃-Rest, besonders bevorzugt für einen Methyl- oder Ethyl-Rest, steht und die Reste R² bis R⁶ die vorstehend genannte Bedeutung haben, gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

Ebenfalls bevorzugt kommen solche 2,5-Diaminomethyl-1 H-pyrrole der allgemeinen Formel I für den Einsatz in den erfindungsgemäßen Arzneimitteln in Betracht, worin R² für einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, fünf- oder sechsgliedrigen Aryl- oder Heteroarylrest oder einen über eine ggf. substituierte C₁₋₃-Alkylen-Gruppe gebundenen, ggf. wenigstens einfach substituierten, fünf- oder sechsgliedrigen Aryl- oder Heteroarylrest, vorzugsweise für einen linearen oder verzweigten, gesättigten aliphatischen C₁₋₃-Rest, besonders bevorzugt für einen Methyl- oder Ethyl-Rest, steht und die Reste R¹ und R³-R⁶ die vorstehend genannte Bedeutung haben, gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

Des weiteren kommen bevorzugt solche 2,5-Diaminomethyl-1 H-pyrrole der allgemeinen Formel für den Einsatz in den erfindungsgemäßen Arzneimitteln in Betracht, worin die Reste R¹ und R² gemeinsam mit dem sie verbindenden Stickstoffatom als Ringglied einen gesättigten oder ungesättigten, ggf. wenigstens einfach substituierten, fünf- oder sechsgliedrigen cycloaliphatischen Rest bilden, der ggf. wenigstens ein weiteres Heteroatom ausgewählt aus der Gruppe bestehend aus N, O und S als Ringglied aufweist, vorzugsweise gemeinsam mit dem sie verbindenden Stickstoffatom als Ringglied einen gesättigten, ggf. Sauerstoff als weiteres Ringglied aufweisenden, fünf- oder sechsgliedrigen cycloaliphatischen Rest bilden, besonders bevorzugt gemeinsam für einen (CH₂)₄-, (CH₂)₅- oder (CH₂)₂-O-(CH₂)₂-Rest stehen, der mit dem sie verbindenden Stickstoffatom als Ringglied einen Heterocyclus bildet und jeweils die Reste R³ bis R⁶ die vorstehend genannte Bedeutung haben, gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

Weiterhin bevorzugt enthalten die erfindungsgemäßen Arzneimittel eine oder mehrere substituierte 2,5-Diaminomethyl-1 H-pyrrole der allgemeinen Formel I, worin R³ für einen unsubstituierten oder wenigstens einfach substituierten, fünf- oder sechsgliedrigen Aryl- oder Heteroarylrest oder für eine Arylester-, Heteroarylester- oder Alkylester-Gruppe, vorzugsweise für einen ggf. wenigstens einfach substituierten Phenylrest oder eine Alkylester-Gruppe mit C₁₋₃, vorzugsweise mit C₁₋C₂, im Alkylteil, steht, und die Reste R¹, R² und R⁴ bis R⁶ die vorstehend genannte Bedeutung haben, gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

Des weiteren sind solche substituierte 2,5-Diaminomethyl-1 H-pyrrole der allgemeinen Formel I in den erfindungsgemäßen Arzneimitteln bevorzugt, worin R⁴ und R⁵, gleich oder verschieden, jeweils für Wasserstoff, einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, 5- oder 6-gliedrigen Aryl- oder Heteroarylrest oder für einen über eine ggf. substituierte C₁₋₃-Alkylen-Gruppe gebundenen, ggf. wenigstens einfach substituierten 5- oder 6-gliedrigen Aryl- oder Heteroarylrest, vorzugsweise für Wasserstoff, einen linearen oder verzweigten, gesättigten aliphatischen C₁₋₃-Rest, besonders bevorzugt für eine Methyl- oder Ethylgruppe, stehen und die Reste R¹ bis R³ und R⁶ die vorstehend genannte Bedeutung haben, gegebenenfalls in Form, ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

Des weiteren kommen bevorzugt solche 2,5-Diaminomethyl-1 H-pyrrole der allgemeinen Formel für den Einsatz in den erfindungsgemäßen Arzneimitteln in Betracht, worin die Reste R⁴ und R⁵ gemeinsam mit dem sie verbindenden Stickstoffatom als Ringglied einen gesättigten oder ungesättigten, ggf. wenigstens einfach substituierten, fünf-, sechs- oder siebengliedrigen cycloaliphatischen Rest bilden, der ggf. wenigstens ein weiteres Heteroatom ausgewählt aus der Gruppe bestehend aus N, O und S als Ringglied aufweist, vorzugsweise gemeinsam mit dem sie verbindenden Stickstoffatom als Ringglied einen gesättigten, ggf. Sauerstoff als weiteres Ringglied aufweisenden, fünf- oder sechsgliedrigen cycloaliphatischen Rest bilden, besonders bevorzugt gemeinsam für einen (CH₂)₄-, (CH₂)₅- oder (CH₂)₂-O-(CH₂)₂-Rest stehen, der mit dem sie verbindenden Stickstoffatom als Ringglied einen Heterocyclus bildet und jeweils die Reste R¹ bis R³ und R⁶ die vorstehend genannte Bedeutung haben, gegebenenfalls in Form, ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

Weiterhin bevorzugt enthalten die erfindungsgemäßen Arzneimittel ein oder mehrere substituierte 2,5-Diaminomethyl-1 H-pyrrole der allgemeinen Formel I, worin der Rest R⁶ für einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden, cycloaliphatischen C₃₋₈-Rest, einen ggf. wenigstens einfach substituierten, 5- oder 6-gliedrigen Aryl- oder Heteroarylrest oder einen über eine ggf. substituierte C₁₋₃-Alkylen-Gruppe gebundenen, ggf. wenigstens einfach substituierten, 5- oder 6-gliedrigen Aryl- oder Heteroarylrest, vorzugsweise für einen linearen oder verzweigten, gesättigten aliphatischen C₁₋₃-Rest, besonders bevorzugt für eine Methylgruppe, steht, und die Reste R¹ bis R⁵ die vorstehend genannte Bedeutung haben, gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

Sofern einer der vorstehend genannten Reste R¹ bis R⁶ für einen aliphatischen oder cycloaliphatischen Rest steht, der einfach oder mehrfach substituiert ist, können die Substituenten vorzugsweise ausgewählt werden aus der Gruppe bestehend aus Halogen, C₁₋₆-Alkoxy, C₁₋₆-Alkyl, Hydroxy, CN, CF₃, CHF₂, CH₂F und ggf. wenigstens einfach substituiertem Phenyl, besonders bevorzugt aus der Gruppe bestehend aus F, Cl, Br, Hydroxy und OCH₃. Sofern der Phenylsubstituent selbst einfach oder mehrfach substituiert ist, können dessen Substituenten bevorzugt ausgewählt werden aus der Gruppe bestehend aus Hydroxy, CN, CF₃, CHF₂, CH₂F, C₁₋₆-Alkyl und C₁₋₆-Alkoxy.

Sofern die vorstehend genannten Reste R¹ bis R⁶ eine einfach oder mehrfach substituierte Alkylen-Gruppe aufweisen, können deren Substituenten vorzugsweise ausgewählt werden aus der Gruppe bestehend aus Halogen, C₁₋₆-Alkoxy, C₁₋₆-Alkyl, Hydroxy, CN, CF₃, CHF₂, CH₂F und ggf. wenigstens einfach substituiertem Phenyl, besonders bevorzugt aus der Gruppe bestehend aus F, Cl, Br, Hydroxy und OCH₃. Sofern der Phenylsubstituent selbst einfach oder mehrfach substituiert ist, können dessen Substituenten bevorzugt ausgewählt werden aus der Gruppe bestehend aus Hydroxy, CN, CF₃, CHF₂, CH₂F, C₁₋₆-Alkyl und C₁₋₆-Alkoxy.

Weist einer der vorstehend genannten Reste R¹ bis R⁶ einen einfach oder mehrfach substituierten Aryl- oder Heteroaryl-Rest auf, so können die entsprechenden Substituenten vorzugsweise ausgewählt werden aus der Gruppe bestehend aus Halogen, Hydroxy, CN, CF₃, CHF₂ und CH₂F, NO₂, NH₂, C₁₋₆-Alkoxy, C₁₋₆-Alkyl und ggf. wenigstens einfach substituiertem Phenyl, besonders bevorzugt aus der Gruppe bestehend aus F, Cl, Br, Hydroxy, OCH₃ und CH₃. Sofern der Phenylsubstituent selbst einfach oder mehrfach substituiert ist, können dessen Substituenten bevorzugt ausgewählt werden aus der Gruppe bestehend aus Hydroxy, CN, CF₃, CHF₂, CH₂F, C₁₋₆-Alkyl und C₁₋₆-Alkoxy.

Sofern einer der vorstehend genannten Reste R¹ bis R⁶ für einen cycloaliphatischen Rest mit wenigstens einem Heteroatom oder für einen Heteroarylrest steht, können die Heteroatome, sofern nicht anders angegeben, bevorzugt ausgewählt werden aus der Gruppe bestehend aus Sauerstoff, Stickstoff und Schwefel.

Geeignete aliphatische Reste, die ggf. einfach oder mehrfach substituiert sind, können beispielsweise ausgewählt werden aus der Gruppe bestehend aus Methyl, Ethyl, n-Propyl, iso-Propyl, n-Butyl, sec-Butyl, tert.-Butyl, n-Pentyl, iso-Pentyl, neoPentyl, n-Hexyl, 2-Hexyl, n-Heptyl, n-Octyl, Vinyl, Ethinyl, Propenyl, Propinyl, Butenyl, Butinyl, Pentenyl, Pentinyl, Hexenyl, Hexinyl, Heptyl, Heptinyl, Octenyl und Octyl.

Geeignete cycloaliphatischen Reste, die ggf. einfach oder mehrfach substituiert sind und/oder ggf. wenigstens ein Heteroatom aufweisen, können beispielsweise ausgewählt werden aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Pyrrolidinyl, Piperidinyl, Piperazinyl und Morpholinyl.

Geeignete Alkylen-Gruppen, die ggf. einfach oder mehrfach substituiert sind, können beispielsweise ausgewählt werden aus der Gruppe bestehend aus Methylen -(CH₂)-, Ethylen -(CH₂)₂-, Propylen -(CH₂)₃-, Butylen -(CH₂)₄-, Pentylen -(CH₂)₅- und Hexylen -(CH₂)₆-, jeweils ggf. auch verzweigt.

Geeignete Aryl-Reste, die ggf. einfach oder mehrfach substituiert sind, sind beispielsweise Phenyl oder Naphthyl, vorzugsweise Phenyl.

Geeignete Heteroaryl-Reste, die ggf. einfach oder mehrfach substituiert sind, können beispielsweise ausgewählt werden aus der Gruppe bestehend aus Pyrrolyl, Furyl, Thienyl, Pyrazolyl, Imidazolyl, Pyridinyl, Pyridazinyl und Pyrimidinyl.

Ganz besonders bevorzugt enthalten die erfindungsgemäßen Arzneimittel wenigstens ein substituiertes 2,5-Diaminomethyl-1 H-pyrrol der allgemeinen Formel I ausgewählt aus der Gruppe bestehend aus
[(2-Methoxyphenyl)-(1-methyl-5-piperidin-1-ylmethyl-1H-pyrrol-2-yl)-methyl]-dimethylamin,
[(2-Methoxyphenyl)-(1-methyl-5-pyrrolidin-1-ylmethyl-1H-pyrrol-2-yl)-methyl]dimethylamin,
Dimethyl-[(1-methyl-5-piperidin-1-ylmethyl-1 H-pyrrol-2-yl)-o-tolyl-methyl]amin,
[(2-Bromphenyl)-(1-methyl-5-piperidin-1-ylmethyl-1H-pyrrol-2-yl)-methyl]-dimethylamin,
[(4-Bromphenyl)-(1-methyl-5-piperidin-1-ylmethyl-1H-pyrrol-2-yl)-methyl]-dimethylamin,
[(4-Fluorphenyl)-(1-methyl-5-morpholin-4-ylmethyl-1H-pyrrol-2-yl)-methyl]-dimethylamin,
[5-(Dimethylaminophenylmethyl)-1-methyl-1 H-pyrrol-2-ylmethyl]-diethylamin,
1-Methyl-5-piperidin-1-ylmethyl- 1H-pyrrol-2-yl)piperidin-1-yl-essigsäureethylester,
(1-Methyl-5-piperidin-1-ylmethyl-1 H-pyrrol-2-yl)morpholin-4-yl-essigsäureethylester,
(5-Diethylaminomethyl-1-methyl-1 H-pyrrol-2-yl)morpholin-4-yl-essigsäureethylester,
(5-Diethylaminomethyl-1-methyl-1 H-pyrrol-2-yl)-pyrrolidin-1-yl-essigsäureethylester,
(1-Methyl-5-morpholin-4-ylmethyl-1 H-pyrrol-2-yl)-piperidin-1-yl-essigsäureethylester
und
(1 -Methyl-5-morpholin-4-ylmethyl-1 H-pyrrol-2-yl)-pyrrolidin-1-yl-essigsäureethylester,
gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

Die substituierten 2,5-Diaminomethyl-1 H-pyrrole der allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Die substituierten 2,5-Diaminomethyl-1 H-pyrrolverbindungen der allgemeinen Formel I lassen sich durch die Umsetzung von Verbindungen der nachstehenden allgemeinen Formel II 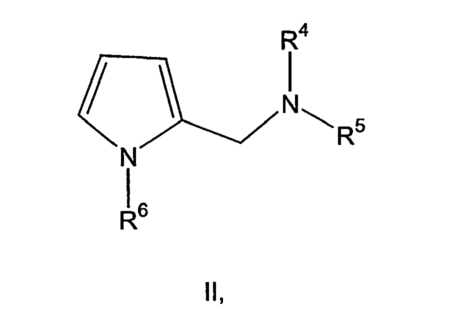 worin die Reste R⁴, R⁵ und R⁶ die vorstehend genannte Bedeutung haben, nach üblichen, dem Fachmann bekannten Methoden, vorzugsweise in einem geeigneten Lösungsmittel, wie z.B. CH₂Cl₂, CH₃CN, Dimethylformamid (DMF) oder Mischungen aus wenigstens zwei dieser Lösungsmittel, bei Raumtemperatur (ca. 20-25°C) mit einem Iminiumsalz der allgemeinen Formel III 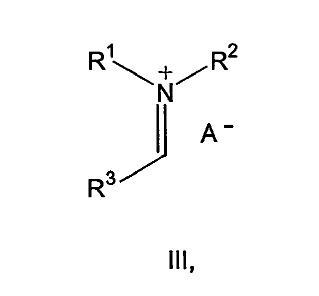 worin R¹, R² und R³ die vorstehend genannte Bedeutung haben und A⁻ für ein geeignetes Anion, vorzugsweise für Cl-, AlCl₄⁻, Br⁻, I⁻ oder CF₃-SO3⁻ (Triflat-Anion) steht, zu einer substituierten 2,5-Diaminomethyl-1 H-pyrrolverbindung der allgemeinen Formel I und ggf. Reinigung und Isolierung nach üblichen, dem Fachmann bekannten Methoden erhalten.

Die Verbindungen der allgemeinen Formel 11 können nach üblichen, dem Fachmann bekannten Methoden_{;} beispielsweise aus den am Markt käuflich erhältlichen Reagenzien der allgemeinen Formel IV 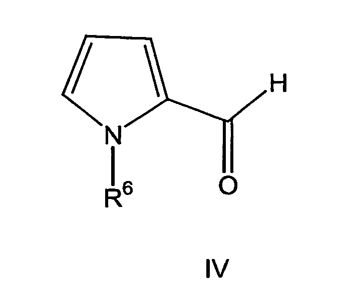 hergestellt werden, wie z.B. in A.F. Abdel-Magid et al., Journal of Organic Chemistry, 1996, 61, Seiten 3849-3862 beschrieben.

Die Imminiumsalze der allgemeinen Formel III können ebenfalls nach üblichen, dem Fachmann bekannten Verfahren, beispielsweise aus den entsprechenden Aminalen der nachstehenden allgemeinen Formel V 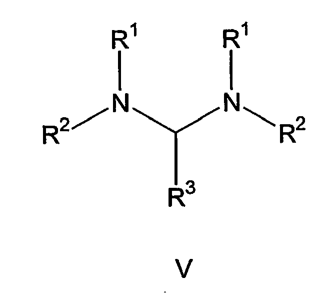 erhalten, wie beispielsweise in D. Seebach et al., Helv. Chim. Acta 1988, 71, Seiten 1999-2021 und N. Risch et al., Synthesis 1998, 11, Seiten 1609-1614 beschrieben.

Auch die Herstellung der Aminale der allgemeinen Formel V kann nach literaturbekannten Methoden erfolgen, wie z.B. D. Seebach et al., Helv. Chim. Acta 1988, 71, Seiten 1999-2021 und N. Risch et al., Synthesis 1998, 11, Seiten 1609-1614 beschrieben.

Die substituierten 2,5-Diaminomethyl-1 H-pyrrole der allgemeinen Formel I sowie entsprechende Stereoisomere können sowohl in Form ihrer freien Basen, ihrer freien Säuren wie auch in Form entsprechender Salze isoliert werden.

Die freien Basen der jeweiligen 2,5-Diaminomethyl-1 H-pyrrole der allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden physiologisch verträglichen Salze überführt werden.

Die freien Basen der jeweiligen 2,5-Diaminomethyl-1 H-pyrrole der allgemeinen Formel I sowie entsprechender Stereoisomere können bevorzugt durch Versetzen der in einem geeigneten organischen Lösungsmittel, wie z.B. Butan-2-on (Methylethylketon), gelösten Verbindungen der allgemeinen Formel 1 oder entsprechender Stereoisomere als freie Basen mit Trimethylsilylchlorid (TMSCI) in die entsprechenden Hydrochloride übergeführt werden.

Die freien Basen der jeweiligen 2,5-Diaminomethyl-1 H-pyrrole der allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze übergeführt werden.

Entsprechend können die freien Säuren der jeweiligen 2,5-Diaminomethyl-1 H-pyrrole der allgemeinen Formel I sowie entsprechender Stereoisomere durch Umsetzung mit einer geeigneten Base in die entsprechenden physiologisch verträglichen Salze überführt werden.

Die 2,5-Diaminomethyl-1 H-pyrrole der allgemeinen Formel I und entsprechende Stereoisomere können ggf., ebenso wie die entsprechenden Säuren, die entsprechenden Basen oder Salze dieser Verbindungen, auch in Form ihrer Solvate, vorzugsweise ihrer Hydrate, erhalten werden.

Sofern die substituierten 2,5-Diaminomethyl-1 H-pyrrole der allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Ein weiterer Gegenstand der vorliegenden Erfindung ist die Verwendung einer oder mehrerer substituierter 2,5-Diaminomethyl-1 H-pyrrole der allgemeinen Formel I, gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate, zur Herstellung eines Arzneimittels zur Bekämpfung von Schmerzen, vorzugsweise chronischen Schmerzen und/oder akuten Schmerzen und/oder neuropathischen Schmerzen, zur Behandlung von Entzugserscheinungen, Gedächtnis-Störungen, neurodegenerativen Erkrankungen, vorzugsweise Morbus Parkinson und/oder Morbus Huntington und/oder Morbus Alzheimer, Epilepsie, Störungen des cardiovaskulären Systems, Wasserspeicher-Krankheiten, intestinaler Motilität (Diarrhoe), Harninkontinenz, Anorexie, Pruritus, Depressionen, Tinnitus, sexueller Dysfunktionen, vorzugsweise erektiler Dysfunktionen, Atemwegserkrankungen, oder zur Diurese, zur Unterdrückung des Miktionsreflexes, zur Anxiolyse, zur Regulation des Elektrolyt-Haushaltes, zur Beeinflussung des cardiovaskulären Systems, vorzugsweise zur Vasodilatation der Arterien, zur Regulation, vorzugsweise Stimulation, der Nahrungsaufnahme, zur Reduzierung des Suchtpotentials von Opioiden, insbesondere von Morphin, zur Modulation der Bewegungsaktivität, zur Beeinflussung der Wirkung von µ-Agonisten, insbesondere Morphin.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben einem oder mehreren substituierten 2,5-Diaminomethyl-1 H-pyrrolen der allgemeinen Formel I, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes, oder in Form ihres Solvates, insbesondere des Hydrates, enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekopstituierbare Trockenzubereitungen sowie Sprays.

Substituierte 2,5-Diaminomethyl-1 H-pyrrole der allgemeinen Formel I, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes, oder in Form ihres Solvates, insbesondere des Hydrates, in einem Depot in gelöster Form oder in einem Pflaster, gegebenenfalls unter Zusatz von die Hautpenetration fördemden Mitteln, sind geeignete perkutane Applikationszubereitungen.

Oral oder perkutan anwendbare Zubereitungsformen können die jeweiligen substituierten 2,5-Diaminomethyl-1 H-pyrrole der allgemeinen Formel I, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes, oder in Form ihres Solvates, insbesondere des Hydrates, auch verzögert freisetzen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind.

Die an den Patienten zu verabreichende Menge des jeweiligen substituierten 2,5-Diaminomethyl-1 H-pyrrols der allgemeinen Formel 1, gegebenenfalls in Form des Racemates, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form der Säure oder Base oder in Form des Salzes, insbesondere eines physiologisch verträglichen Salzes, oder in Form des Solvates, insbesondere des Hydrates, kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.05 bis 5 mg/kg Körpergewicht des Patienten wenigstens eines substituierten 2,5-Diaminomethyl-1 H-pyrrols der allgemeinen Formel I, gegebenenfalls in Form seines Racemates, seines reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form seiner Säure oder seiner Base oder in Form seines Salzes, insbesondere eines physiologisch verträglichen Salzes, oder in Form ihres Solvates, insbesondere des Hydrates, appliziert.

### Pharmakologische Methoden:

### a) Methode zur Bestimmung der Affinität zu dem ORL-1-Rezeptor

Die Affinität des jeweiligen substituierten 2,5-Diaminomethyl-1 H-pyrrols der allgemeinen Formel I zu dem ORL-1-Rezeptor wurde in einem Rezeptorbindungsassay mit ³H-Nociceptin/Orphanin FQ mit Membranen von rekombinanten CHO-ORL1 Zellen bestimmt, wie von Ardati et al. in Mol. Pharmacol., 51, 1997, Seiten 816-824 beschrieben. Die entsprechende Literaturbeschreibung wird hiermit als Referenz eingeführt und gilt als Teil der Offenbarung.

Die Konzentration von ³H-Nociceptin/Orphanin FQ betrug bei diesen Versuchen 0,5 nM. Die Bindungsassays wurden mit jeweils 20 µg Membranprotein je 200 µl Ansatz in 50 mM Hepes, pH 7,4,10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg, Deutschland), durch einstündige Inkubation des Ansatzes bei Raumtemperatur (ca. 20-25 °C) und anschließende Messung im Szintillationscounter Trilux (Wallac, Finnland), bestimmt.

### b) Methode zur Bestimmung der Affinität zum humanen µ-Opiatrezeptor

Die Rezeptoraffinität zum humanen µ-Opiatrezeptor wird in einem homogenen Ansatz in Mikrotiterplatten bestimmt. Hierzu werden Verdünnungsreihen des jeweils zu prüfenden substituierten 2,5-Diaminomethyl-1 H-pyrrols mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wird 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wird zusätzlich 25 µmol/l Naloxon zugegeben. Nach Beendigung der neunzigminütigen Inkubationszeit werden die Mikrotiterplatten für 20 Minuten bei 1000 g abzentrifugiert und die Radioaktivität in einem ß-Counter (Microbeta-Trilux, Firma PerkinElmer Wallac, Freiburg, Deutschland) vermessen. Es wird die prozentuale Verdrängung des radioaktiven Liganden aus seiner Bindung zum humanen µ-Opiatrezeptor bei einer Konzentration der Prüfsubstanzen von 1 µmol/l bestimmt und als Prozent Hemmung der spezifischen Bindung angegeben. Ausgehend von der prozentualen Verdrängung durch unterschiedliche Konzentrationen der zu prüfenden Verbindungen der allgemeinen Formel I werden IC₅₀ Hemmkonzentrationen berechnet, die eine 50-prozentige Verdrängung des radioaktiven Liganden bewirken. Durch Umrechnung mittels der Cheng-Prusoff-Beziehung werden Kᵢ-Werte für die Prüfsubstanzen erhalten.

### c) Methode zur Bestimmung der Noradrenalin- und der 5HT-Uptake-Inhibierung:

Für in vitro Studien wurden Synaptosomen aus Rattenhimarealen frisch isoliert, wie in der Veröffentlichung "The isolation of nerve endings from brain" von E.G. Gray und V.P. Whittaker, J. Anatomy 96, Seiten 79-88, 1962, beschrieben. Die entsprechende Literaturbeschreibung wird hiermit als Referenz eingeführt und gilt als Teil der Offenbarung.
Das Gewebe (Hypothalamus für die Bestimmung der Noradrenalin-Uptake-Inhibierung und Mark und Pons für die Bestimmung der 5HT-Uptake-Inhibierung) wurde in eisgekühlter 0,32 M Sucrose (100 mg Gewebe / 1mL) in einem Glas-Homogenisierer mit Teflonstößel homogenisiert, indem fünf volle Auf-und Abschläge bei 840 Umdrehungen /Minute benutzt wurden.

Das Homogenat wurde bei 4°C für 10 Minuten bei 1000 g zentrifugiert. Nach anschließender Zentrifugierung bei 17000 g für 55 Minuten erhält man die Synaptosomen (P₂-Fraktion), die in 0,32 M Glucose (0,5 mL/100 mg des ursprünglichen Gewichts) noch einmal suspendiert wurden.

Der jeweilige Uptake wurde in einer 96-well Mikrotiterplatte gemessen. Das Volumen betrug 250 µl und die Inkubation erfolgte bei Raumtemperatur (ca. 20-25 °C) unter O₂ Atmosphäre.

Die Inkubationszeit betrug 7,5 Minuten für [³H]-NA und 5 Minuten für [³H]-5-HT. Anschließend wurden die 96 Proben durch eine Unifilter GF/B® Mikrotiterplatte (Packard) filtriert und mit 200 mL inkubierten Puffer mit Hilfe eines "Brabdel Cell-Harvester MPXRI-96T" gewaschen. Die Unifilter GF/B Platte wurde bein 55°C 1 h getrocknet. Im Anschluß wurde die Platte mit einem Back seal® (Packard) verschlossen und 35 µl Szintilationsflüssigkeit pro Weil (Ultima Gold® , Packard) versetzt. Nach dem Verschließen mit einem top seal® (Packard) wurde, nach der Einstellung des Gleichgewichts (etwa 5 h), die Radioaktivität in einem "Trilux 1450 Microbeta" (Wallac) bestimmt.

Folgende Kenndaten wurden für den NA-Transporter ermittelt:
NA-Uptake : Km = 0,32 ± 0,11 µM

Die Menge des bei der vorstehenden Bestimmung eingesetzten Proteins entsprach den aus der Literatur bekannten Werten, wie z.B. in "Protein measurement with the folin phenol reagent", Lowry et al., J. Biol. Chem., 193, 265-275, 1951 beschrieben. Eine detaillierte Methodenbeschreibung kann auch der Literatur, beispielsweise aus M.Ch. Frink, H.-H. Hennies, W. Engelberger, M. Haurand und B. Wilffert (1996) Arzneim.-Forsch./Drug Res. 46 (lll), 11, 1029-1036, entnommen werden. Die entsprechenden Literaturbeschreibungen werden hiermit als Referenz eingeführt und gelten als Teil der Offenbarung.

### d) Untersuchung auf analgetische Wirksamkeit im Writhing-Test

Die Untersuchung der substituierten 2,5-Diaminomethyl-1 H-pyrrole der allgemeinen Formel I auf analgetische Wirksamkeit wurde im Phenylchinon-induzierten Writhing an der Maus, modifiziert nach I.C. Hendershot und J. Forsaith (1959) J. Pharmacol. Exp. Ther. 125, 237-240 durchgeführt. Die entsprechende Literaturbeschreibung wird hiermit als Referenz eingeführt und gilt als Teil der Offenbarung.

Dazu wurden männliche NMRI-Mäuse mit einem Gewicht von 25 bis 30 g verwendet. Gruppen von 10 Tieren pro Verbindungsdosis erhielten 10 Minuten nach intravenöser Gabe der zu untersuchenden Verbindungen 0,3 ml/Maus einer 0,02%igen wäßrigen Lösung von Phenylchinon (Phenylbenzochinon, Fa. Sigma, Deisenhofen, Deutschland; Herstellung der Lösung unter Zusatz von 5 Gew.-% Ethanol und Aufbewahrung im Wasserbad bei 45°C) intraperitoneal appliziert. Die Tiere wurden einzeln in Beobachtungskäfige gesetzt. Mit Hilfe eines Drucktastenzählers wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten hatten. Alle Verbindungen wurden in der Standarddosierung von 10 mg/kg getestet.

### Beispiele:

Die Messung der NMR-Spektren erfolgte auf einem Gerät vom Typ Bruker DPX 300 (für 300 MHz-Spektren) und vom Typ Bruker DRX 600 (für 600 MHz-Spektren).

Die jeweiligen Chemikalien und Lösungsmittel wurden kommerziell von den üblichen Herstellern erworben.

### A)

### Allgemeine Synthesevorschrift zur Herstellung von Verbindungen der allgemeinen Formel II:

Der jeweilige Pyrrolcarbaldehyd (90 mmol) der allgemeinen Formel IV wurde in 600 ml Tetrahydrofuran gelöst und nacheinander mit dem entsprechenden Amin (90 mmol) und Natriumborhydridtriacetat (NaBH(OAc)₃ (126 mmol) versetzt. Nach 20 Stunden Rühren bei Raumtemperatur (ca. 20-25 °C) wurde das Reaktionsgemisch am Rotationsverdampfer eingeengt. Der so erhaltene Rückstand wurde in 500 ml Wasser und 200 ml Diethylether aufgenommen und mit Eisessig (30 ml) auf pH 4-5 eingestellt. Es wurde dreimal mit je 100 ml Diethylether extrahiert. Anschließend wurde die wäßrige Phase mit gesättigter Natriumhydrogencarbonat-Lösung auf pH 8 eingestellt und fünfmal mit je 100 ml Diethylether extrahiert. Die organische Phase wurde über Magnesiumsulfat getrocknet und am Rotationsverdampfer eingeengt.

Die nach der vorstehenden allgemeinen Synthesevorschrift hergestellten Verbindungen der allgemeinen Formel II sind in der nachfolgenden Tabelle 1 wiedergegeben:

**Tabelle 1:**

| **Verbindung** | **R**^{**4**} | **R**^{**5**} | **R**^{**6**} |
|---|---|---|---|
| II-1 | -(CH₂)₂-O-(CH₂)₂- | | CH₃ |
| II-2 | N-Methylpiperazinyl | | CH₃ |
| II-3 | -(CH₂)₅- | | CH₃ |
| II-4 | -(CH₂)₄- | | CH₃ |
| II-5 | CH₂CH₃ | CH₂CH₃ | CH₃ |
| II-6 | (CH₂)₂-Phenyl | H | CH₃ |
| II-7 | CH₂-Phenyl | CH₃ | CH₃ |
| II-8 | Cyclopropyl | H | CH₃ |
| II-9 | Cyclohexyl | H | CH₃ |
| II-10 | Cyclohexyl | CH₃ | CH₃ |
| II-11 | -(CH₂)₆- | | CH₃ |
| II-12 | (CH₂)₃CH₃ | H | CH₃ |
| II-13 | (CH₂)₃CH₃ | CH₃ | CH₃ |
| II-14 | Allyl | Allyl | CH₃ |
| II-15 | CH₂-ortho-OCH₃-Phenyl | H | CH₃ |

Die Struktur der Verbindungen II-1 bis II-15 wurde jeweils mit Hilfe der ¹H-NMR-Spektroskopie bestimmt. Im folgenden sind die chemischen Verschiebungen einiger ausgewählter Verbindungen wiedergegeben.

### II-2)

### 1-Methyl-4-(1-methyl-1 H-pyrrol-2-ylmethyl)-piperazin

δ (DMSO, 300 MHz): 2,26 (s, 3 H, C*H*₃N(CH₂)₄N); 2,30 - 2,59 (m, 8 H, CH₃N(C*H*₂)₄N); 3,37 - 3,48 (m, 2 H, -C*H*₂-N-); 3,62 (s, 3 H, N-CH₃); 5,97 - 6,02 (m, 2 H, N(CH₃)-]-C*H*C*H*CHC-[); 6,54 - 6,60 (m, 1 H, N(CH₃)-]-CHCHC*H*C-[).

### II-6)

### (1-Methyl-1 H-pyrrol-2-ylmethyl)-phenethyl-amin

δ (DMSO, 300 MHz): 2,66 - 2,78 (m, 4 H, N-C*H*₂C*H*₂Ph); 3,52 (s, 3 H, N-CH₃); 3,63 (s, 2 H, -C*H*₂-N-); 5,85 (s, 2 H, N(CH₃)-]-C*H*C*H*CHC-[); 6,58 (s, 1 H, N(CH₃)]-CHCHC*H*C-[); 7,13 - 7,32 (m, 5 H, Ph).

### II-7)

### Benzyl-methyl-(1-methyl-1H-pyrrol-2-ylmethyl)-amin

δ (DMSO, 300 MHz): 2,11 (s, 3 H, N(C*H*₃)CH₂Ph); 3,41 - 3,44 (m, 2 H, N(CH₃)C*H*₂Ph); 3,45 - 3,48 (m, 2 H, -C*H*₂-N-); 5,99 - 6,03 (m, 2 H, N(CH₃)-]-C*H*C*H*CHC-[); 6,53 - 6,58 (m, 1 H, N(CH₃)-]-CHCHC*H*C-[); 7,17 - 7,32 (m, 5 H, Ph).

### II-8)

### Cyclopropyl-(1-methyl-1H-pyrrol-2-ylmethyl)-amin

δ (DMSO, 300 MHz): 0,29 - 0,48 (m, 4 H, -CH₂CH₂CH-); 1,66 (s, 1 H, NH); 2,12 - 2,22 (m, 1 H, -CH₂CH₂CH-); 3,58 (s, 3 H, N-CH₃); 3,74 - 3,81 (m, 2 H, -CH₂-N-); 5,99 - 6,04 (m, 2 H, N(CH₃)-]-CHCHCHC-[ ); 6,50 - 6,55 (m, 1 H, N(CH₃)-]-CHCHCHC-[).

### II-10)

### Cyclohexyl-methyl-(1-methyl-1H-pyrrol-2-ylmethyl)-amin

δ (DMSO, 300 MHz): 1,00 - 1,35 (m, 5 H, NCH(CH₂CH₂)₂CH₂); 1,52 -1,69 (m, 1 H, NCH(CH₂CH₂)₂CH₂); 1,70 -1,90 (m, 4 H, NCH(CH₂CH₂)₂CH₂); 2,13 (s, 3 H, N-CH₃); 2,35 - 2,46 (m, 1 H, NCH(CH₂CH₂)₂CH₂); 3,43 - 3,55 (m, 2 H, -CH₂-N-); 3,61 (s, 3 H, N-CH₃); 5,92 - 5,97 (m, 1 H, N(CH₃)-]-CHCHCHC-[); 5,98 - 6,03 (m, 1 H, N(CH₃)-]-C*H*C*H*CHC-[); 6,52 - 6,57 (s, 1 H, N(CH₃)-]-CHCHC*H*C-[).

### II-11)

### 1-(1-Methyl-1H-pyrrol-2-ylmethyl)-azepan

δ (DMSO, 300 MHz): 1,52 -1,65 (m, 8 H, N(CH₂)₂(CH₂)₂(CH₂)₂); 2,51 - 2,60 (m, 4 H, N(CH₂)₂(CH₂)₂(CH₂)₂); 3,49 - 3,52 (m, 2 H, -CH₂-N-); 3,62 (s, 3 H, N-CH₃); 5,91 - 5,98 (m, 1 H, N(CH₃)-]-C*H*C*H*CHC-[); 5,99 - 6,04 (m, 1 H, N(CH₃)-]-C*H*C*H*CHC-[); 6,53 - 6,61 (m, 1 H, N(CH₃)-]-CHCHC*H*C-[).

### II-13)

### Butyl-(1-methyl-1H-pyrroi-2-ylmethyl)-amin

δ (DMSO, 300 MHz): 0,88 (t, 3 H, J = 7,2 Hz, CH₃CH₂CH₂CH₂N); 1,23 -1,37 (m, 2 H, CH₃CH₂CH₂CH₂N); 1,38 -1,52 (m, 2 H, CH₃CH₂CH₂CH₂N); 2,11 (s, 3 H, N-CH₃); 2,31 (d, 1 H, J = 7,5 Hz, CH₃CH₂CH₂CH₂N); 2,33 (d, 1 H, J = 7,2 Hz, CH₃CH₂CH₂C*H*₂N); 3,34 - 3,39 (m, 2 H, -CH₂-N-); 3,62 (s, 3 H, N-CH₃); 5,93 - 5,97 (m, 1 H, NCH₂CHCH₂); 5,98 - 6,04 (m, 1 H, N(CH₃)-]-C*H*C*H*CHC-[); 6,52 - 6,58 (m, 1 H, N(CH₃)-]-CHCHCHC-[).

### II-14)

### Diallyl-(1-methyl-1H-pyrrol-2-ylmethyl)-amin

δ (DMSO, 300 MHz): 3,01 - 3,06 (m, 4 H, NC*H*₂CHCH₂); 3,44 - 3,50 (m, 2 H, -C*H*₂₋N-); 3,62 (s, 3 H, N-CH₃); 5,08 - 5,22 (m, 4 H, NCH₂CHC*H*₂); 5,75 - 5,91 (m, 2 H, NCH₂CHCH₂); 5,96 - 6,03 (m, 2 H, N(CH₃)-]-C*H*C*H*CHC-[); 6,55 - 6,57 (m, 1 H, N(CH₃)-]-CHCHC*H*C-[); 7,13 - 7,32 (m, 5 H, Ph).

### II-15)

### (2-Methoxy-benzyl)-(1-methyl-1H-pyrrol-2-ylmethyl)-amin

δ (DMSO, 300 MHz): 2,03 (s, 1 H, NH); 3,60 (s, 3 H, N-CH₃); 3,67 - 3,73 (m, 2 H,-CH₂-N-); 3,81 (s, 5 H, CH₂-2-OMePh, OMe); 6,00 - 6,05 (m, 2 H, N(CH₃)-]-CHCHCHC-[); 6,52 - 6,60 (m, 1 H, N(CH₃)-]-CHCHCHC-[); 6,80 - 6,98 (m, 2 H, 2-OMePh); 7,16 - 7,29 (m, 2 H, 2-OMePh).

Die für die übrigen Verbindungen II-1, II-3 bis II-5, II-9 und II-12 gefundenen Werte der chemischen Verschiebungen entsprachen den jeweils aus der Literatur bekannten Werten.

### B)

### Allgemeine Synthesevorschrift zur Herstellung von Verbindungen der allgemeinen Formel III:

Eine Lösung von einem Äquivalent Acetylchlorid in Diethylether wurde langsam unter Rühren zu der eisgekühlten Lösung bzw. Suspension von einem Äquivalent der jeweiligen Verbindung der allgemeinen Formel V getropft. Anschließend wurde das Reaktionsgemisch für eine Stunde bei Raumtemperatur nachgerührt. Es entstand ein Niederschlag, der unter Stickstoff abgesaugt und anschließend im Ölpumpenvakuum getrocknet wurde. Die so erhaltenen Imminiumsalze der allgemeinen Formel III wurden dann ohne jede weitere Aufreinigung eingesetzt.

Abweichend von dieser Vorschrift wurden die Verbindungen III-7, III-8 und lll-9 nicht isoliert, sondem unmittelbar im nachfolgenden Syntheseschritt eingesetzt.

Die nach der vorstehenden allgemeinen Synthesevorschrift hergestellten Verbindungen der allgemeinen Formel III sind in der nachfolgenden Tabelle 2 wiedergegeben:

**Tabelle 2**

| **Verbindung** | **R**^{**1**} | **R**^{**2**} | **R**^{**3**} |
|---|---|---|---|
| III-1 | CH₃ | CH₃ | ortho-OCH₃-phenyl |
| III-2 | CH₃ | CH₃ | ortho-Methyl-phenyl |
| III-3 | CH₃ | CH₃ | ortho-Brom-phenyl |
| III-4 | CH₃ | CH₃ | para-Brom-phenyl |
| III-5 | CH₃ | CH₃ | para-Fluor-phenyl |
| III-6 | CH₃ | CH₃ | Phenyl |
| III-7 | -(CH₂)₅⁻ | | COOC₂H₅ |
| III-8 | -(CH₂)₂-O(CH₂)₂⁻ | | COOC₂H₅ |
| III-9 | -(CH₂)₄⁻ | | COOC₂H₅ |

### Beispiele 1-7

### C)

### Allgemeine Synthesevorschrift zur Herstellung der Verbindungen gemäß Beispiel 1-7:

Äquimolare Mengen der jeweiligen Verbindungen der allgemeinen Formel II und III wurden in CH₂Cl₂, CH₃CN und/oder DMF gelöst und über Nacht bei Raumtemperatur (ca. 20 bis 25 °C) gerührt. Die so erhaltene Lösung wurde zunächst mit wäßriger Salzsäure angesäuert und nichtbasische Verunreinigungen mit Diethylether extrahiert. Anschließend wurde die wäßrige Phase mit Na₂CO₃-Lösung versetzt und die jeweilige Beispielverbindung mit Diethylether extrahiert. Die so erhaltene etherische Lösung wurde über Magnesiumsulfat getrocknet. Anschließend wurde die jeweilige Verbindung gemäß Beispiel 1-7 mit Hilfe einer ethanolischen Chlorwasserstoff-Lösung in das entsprechende Dihydrochlorid übergeführt und durch Filtration isoliert. Das so erhaltene Salz wurde anschließend durch Waschen mit Ethanol, Tetrahydrofuran, Diethylether oder Aceton gereinigt:

Die jeweils zur Herstellung der Beispielverbindungen eingesetzten Verbindungen der allgemeinen Formeln 11 und 111 sowie das verwendete Lösungsmittel sind in der nachfolgenden Tabelle 3 wiedergegeben.

**Tabelle 3**

| Beispiel | Verbindung der allgemeinen Formel II | Iminiumsalz der allgemeinen Formel III | Lösungsmittel |
|---|---|---|---|
| 1 | II-3 | III-1 | Acetonitril |
| 2 | II-4 | III-1 | Acetonitril |
| 3 | II-3 | III-2 | Dichlormethan |
| 4 | II-3 | III-3 | Dichlormethan |
| 5 | II-3 | III-4 | Dimethylformamid |
| 6 | II-1 | III-5 | Gemisch aus Dimethylformamid und Dichlormethan |
| 7 | II-5 | III-6 | Dimethylformamid |

Die Struktur der Verbindungen gemäß den Beispielen 1-7 wurde jeweils mit Hilfe der ¹H-NMR-Spektroskopie bestimmt. Die gefundenen chemischen Verschiebungen waren wie folgt:

### Beispiel 1:

### [(2-Methoxy-phenyl)-(1-methyl-5-piperidin-1-ylmethyl-1H-pyrrol-2-yl)-methyl]-dimethyl-amin Dihydrochlorid

δ (DMSO, 600 MHz): 1,29 -1,41 (m, 1 H, N(CH₂CH₂)₂CH₂); 1,66 - 1,88 (m, 5 H, N(CH₂CH₂)₂CH₂, N(CH₂CH₂)₂CH₂); 2,68 (d, 3 H, J = 4,5 Hz, N(CH₃)₂); 2,27 (d, 3 H, J = 4,5 Hz, N(CH₃)₂); 2,79 - 2,91 (m, 2 H, N(CH₂CH₂)₂CH₂); 3,27 - 3,35 (m, 2 H, N(CH₂CH₂)₂CH₂); 3,77 (s, 3 H, NCH₃); 3,90 (s, 3 H, OCH₃); 4,22 (dd, 2 H, J = 5,2 Hz, J = 14,4 Hz, CH₂N(CH₂CH₂)₂CH₂); 4,27 (dd, 2 H, J = 4,3 Hz, J = 14,4 Hz, CH₂N(CH2CH₂)₂CH₂); 5,92 (d, 1 H, J = 9,1 Hz; CHN(CH₃)₂); 6,38 (d, 1 H, *J* = 3,8 Hz, N(CH₃)]-CCHCHC-[); 6,60 (d, 1 H, J = 3,8 Hz, N(CH₃)]-CCHCHC-[ ); 7,05 (t, 1 H, J = 7,6 Hz, o-OMePh); 7,10 (d, 1 H, J = 7,6 Hz, o-OMePh); 7,37 (t, 1 H, J = 7,6 Hz, o-OMePh); 7,94 (d, 1 H, J = 7,6 Hz, o-OMePh); 10,31 (s, 1 H, HCl); 11,06 (s, 1 H, HCl).

### Beispiel 2:

### [(2-Methoxy-phenyl)-(1-methyl-5-pyrrolidin-1-ylmethyl-1H-pyrrol-2-yl)-methyl]-dimethyl-amin Dihydrochlorid

δ (DMSO, 600 MHz) = 1,85 -1,92 (m, 2 H, N(CH₂CH₂)₂); 1,95 - 2,07 (m, 2 H, N(CH₂C*H*₂)₂); 2,68 (d, 3 H, J = 4,5 Hz, N(CH₃)₂); 2,72 (d, 3 H, J = 4,5 Hz, N(CH₃)₂); 3,01 - 3,12 (m, 2H, N(C*H*₂CH₂)₂); 3,27 - 3,37 (m, 2 H, N(C*H*₂CH₂)₂); 3,78 (s, 3 H, NC*H*₃); 3,90 (s, 3 H, OCH₃); 4,30 (dd, 1 H, J = 6,1 Hz, J = 14,4 Hz, C*H*₂N(CH₂C*H*₂)₂); 4,40 (dd, 1 H, J = 6,1 Hz, J = 14,4 Hz, CH₂-N(CH₂CH₂)₂); 5,91 (d, 1 H, J = 9,8 Hz, CHN(CH₃)₂); 6,38 (d, 1 H, J = 3,8 Hz, N(CH₃)]-CC*H*C*H*C-[); 6,55 (d, 1 H, J = 3,8 Hz, N(CH₃)]-CC*H*C*H*C-[); 7,05 (t, 1 H, J = 7,6 Hz, o-OMePh); 7,11 (d, 1 H, J = 7,6 Hz, o-OMePh); 7,38 (t, 1 H, J = 7,9 Hz, o-OMePh); 7,91 (d, 1 H, J = 6,8 Hz, o-OMePh); 10,72 (s, 1 H, HCl); 10,89 (s, 1 H, HCl).

### Beispiel 3:

### Dimethyl-[(1-methyl-5-piperidin-1-ylmethyl-1 H-pyrrol-2-yl)-o-tolyl-methyl]-amin Dihydrochlorid

δ (DMSO, 600 MHz): 1,30 -1,41 (m, 1 H, N(CH₂CH₂)₂CH₂); 1,64 -1,90 (m, 5 H, N(CH₂CH₂)₂CH₂, N(CH₂CH₂)₂CH₂); 2,41 (s, 3 H, PhC*H*₃); 2,70 (d, 3 H, J = 3,8 Hz, N(CH₃)₂); 2,71 (d, 3 H, J = 3,8 Hz, N(CH₃)₂); 2,80 - 2,94 (m, 2 H, N(C*H*₂CH₂)₂CH₂); 3,27 - 3,41 (m, 2 H, N(CH₂CH₂)₂CH₂); 3,88 (s, 3 H, NCH₃); 4,20 - 4,31 (m, 2 H, C*H*₂N(CH₂C*H*₂)₂CH₂); 5,80 (d, 1 H, J = 9,0 Hz, CHN(CH₃)₂); 6,36 - 6,42 (m, 1 H, N(CH₃)]-CC*H*C*H*C-[); 6,43 - 6,50 (m, 1 H, N(CH₃)]-CC*H*C*H*C-[); 7,22 - 7,30 (m, 2 H, o-MePh); 7,31 - 7,38 (m, 1 H, o-MePh); 8,18 (d, 1 H, J = 7,6 Hz, o-MePh); 10,43 (s, 1 H, HCl); 11,26 (s, 1 H, HCl).

### Beispiel 4:

### [(2-Bromo-phenyl)-(1-methyl-5-piperidin-1-ylmethyl-1 H-pyrrol-2-yl)-methyl]-dimethylamin Dihydrochlorid

δ (DMSO, 600 MHz): 1,31 -1,40 (m, 1 H, N(CH₂CH₂)₂C*H*₂); 1,65 -1,91 (m, 5 H, N(CH₂CH₂)₂C*H*₂, N(CH₂C*H*₂)₂CH₂); 2,73 (m, 6 H, N(CH₃)₂); 2,80 - 2,95 (m, 2 H, N(C*H*₂CH₂)₂CH₂); 3,23 - 3,47 (m, 2 H, N(C*H*₂CH₂)₂CH₂); 3,92 (s, 3 H, NC*H*₃); 4,23 (dd, 1H, *J* = 5,6 Hz, *J* = 14,7 Hz, C*H*₂-N(CH₂C*H*₂)₂CH₂); 4,81 (dd, 1 H, *J* = 4,1 Hz, *J* = 14,7 Hz, C*H*₂N(CH₂C*H*₂)₂CH₂); 5,90 (d, 1 H, *J* = 9,1 Hz, C*H*N(CH₃)₂); 6,38 - 6,43 (m, 1H, N(CH₃)]-CC*H*C*H*C-[); 6,44 - 6,50 (m, 1 H, N(CH₃)]-CC*H*C*H*C-[); 7,35 (t, 1 H, J = 7,6 Hz, o-BrPh); 7,56 (t, 1 H, J = 7,6 Hz, o-BrPh); 7,70 (d, 1 H, J = 8,3 Hz, o-BrPh); 8,49 (d, 1 H, J = 8,3 Hz, o-BrPh); 10,48 (s, 1 H, HCl); 11,81 (s, 1 H, HCl).

### Beispiel 5:

### [(4-Bromo-phenyl)-(1-methyl-5-piperidin-1-ylmethyl-1 H-pyrrol-2-yl)-methyl]-dimethylamin Dihydrochlorid

δ (DMSO, 600 MHz): 1,30 -1,39 (m, 1 H, N(CH₂CH₂)₂C*H*₂); 1,66 -1,85 (m, 5 H, N(CH₂CH₂)₂C*H*₂, N(CH₂C*H*₂)₂CH₂); 2,63 (d, 3 H, *J* = 4,5 Hz, N(CH₃)₂); 2,73 (d, 3 H, *J* = 4,5 Hz, N(CH₃)₂); 2,78 - 2,92 (m, 2 H, N(C*H*₂CH₂)₂CH₂); 3,28 - 3,38 (m, 2 H, N(C*H*₂CH₂)₂CH₂); 3,72 (s, 3 H, NC*H*₃); 4,23 (dd, 1 H, *J =* 5,3 Hz, *J =* 15,1 Hz, C*H*₂N(CH₂C*H*₂)₂CH₂); 4,26 (dd, 1 H, *J =* 4,5 Hz, *J* =15,1 Hz, C*H*₂N(CH₂C*H*₂)₂CH₂); 5,85 (d, 1 H, *J =* 9,1 Hz, C*H*N(CH₃)₂); 6,43 (d, 1 H, *J =* 3,8 Hz, N(CH₃)]-CC*H*C*H*C-[); 6,84 (d, 1 H, *J* = 3,8 Hz, N(CH₃)]-CC*H*C*H*C-[); 7,66 (d, 2 H, *J* = 8,3 Hz, *p*-BrPh); 7,76 (d, 2 H, *J =* 8,3 Hz, *p*-BrPh); 10,17 (s, 1 H, HCl); 11,46 (s, 1 H, HCl).

### Beispiel 6:

### [(4-Fluoro-phenyl)-(1-methyl-5-morpholin-4-ylmethyl-1 H-pyrrol-2-yl)-methyl]-dimethylamin Dihydrochlorid

δ (DMSO, 600 MHz): 2,45 - 2,56 (m, 4 H, N(CH₂CH₂)₂O); 2,62 (d, 3 H, J = 4,5 Hz, N(CH₃)₂); 2,74 (d, 3 H, J = 4,5 Hz, N(CH₃)₂); 3,01 - 3,14 (m, 2 H, N(CH₂CH₂)₂0); 3,22 - 3,37 (m, 2 H, N(CH₂CH₂)₂0); 3,75 (s, 3 H, NCH₃); 3,78 - 3,88 (m, 2 H, N(CH₂CH₂)₂O); 3,88 - 3,98 (m, 2 H, N(CH₂CH₂)₂O); 4,26 - 4,40 (m, 2 H, CH₂N(CH₂CH₂)₂O); 5,85 (d, 1 H, J = 9,1 Hz, CHN(CH₃)₂); 6,40 - 6,48 (m, 1 H, N(CH₃)]-CCHCHC-[); 6,81 - 6,90 (m, 1 H, N(CH₃)]-CCHCHC-[); 7,23 - 7,33 (m, 2 H, p-FPh); 7,77 - 7,89 (m, 2 H, p-FPh); 10,99 (s, 1 H, HCl); 11,60 (s, 1 H, HCl).

### Beispiel 7:

### [5-(Dimethylamino-phenyl-methyl)-1-methyl-1 H-pyrrol-2-ylmethyl]-diethyl-amin Dihydrochlorid

δ (DMSO, 600 MHz): 1,16 - 1,32 (m, 6 H, N(CH₂C*H*₃)₂); 2,55 - 2,67 (m, 3 H, N(CH₃)₂); 2,67 - 2,79 (m, 3 H, N(CH₃)₂); 2,95 - 3,14 (m, 4 H, N(C*H*₂CH₃)₂); 3,77 (s, 3 H, NC*H*₃); 4,15 - 4,26 (m, 1 H, C*H*₂N(CH₂CH₃)₂); 4,26 - 4,36 (m, 1 H, C*H*₂N(CH₂CH₃)₂); 5,86 (d, 1 H, *J* = 9,1 Hz, C*H*N(CH₃)₂); 6,35 - 6,47 (m, 1 H, N(CH₃)]-CC*H*C*H*C-[); 6,82 - 6,94 (m, 1 H, N(CH₃)]-CC*H*C*H*C-[); 7,30 - 7,52 (m, 3 H, Ph); 7,75 - 7,90 (m, 3 H, Ph); 10,50 (s, 1 H, HCl); 11,83 (s, 1 H, HCl).

### Beispiele 8-13

### D)

### Allgemeine Synthesevorschrift zur Herstellung der beispielgemäßen Verbindungen 8-13:

Die jeweilige Verbindung der allgemeinen Formel III wurde gemäß der vorstehenden allgemeinen Synthesevorschrift B), abweichend davon jedoch in Acetonitril, Dichlormethan oder Dimethylformamid, aus dem entsprechenden Aminal der allgemeinen Formel V in situ hergestellt. Anschließend wurde eine äquimolare Menge der jeweiligen Verbindung der allgemeinen Formel II zugegeben und über Nacht bei Raumtemperatur (ca. 20-25 °C) gerührt. Zur Aufarbeitung wurde zunächst mit wäßriger Salzsäure-Lösung angesäuert und nichtbasische Verunreinigungen mit Ether extrahiert. Anschließend wurde die wäßrige Phase mit Na₂CO₃-Lösung schwach basisch gestellt und die jeweilige Verbindung gemäß Beispiel 8-13 mit Diethylther extrahiert. Nach dem Trocknen über Mg₂SO₄ erhielt man das Produkt, das zur weiteren Aufreinigung mit Hilfe von ethanolischer HCl-Lösung in das Dihydrochlorid übergeführt wurde. Die Aufreinigung erfolgte durch Waschen mit Ethanol, Ether oder Aceton.

Die jeweils zur Herstellung der Beispielverbindungen eingesetzten Verbindungen der allgemeinen Formeln 11 und 111 sowie das verwendete Lösungsmittel sind in der nachfolgenden Tabelle 4 wiedergegeben.

**Tabelle 4:**

| Beispiel | Verbindung der allgemeinen Formel II | Iminiumsalz der allgemeinen Formel III | Lösungsmittel |
|---|---|---|---|
| 8 | II-3 | III-7 | Dichlormethan |
| 9 | II-3 | III-8 | Acetonitril |
| 10 | II-5 | III-8 | Acetonitril |
| 11 | II-5 | III-9 | Dimethylformamid |
| 12 | II-1 | III-7 | Dimethylformamid |
| 13 | II-1 | III-9 | Dimethylformamid |

Die Struktur der Verbindungen gemäß den Beispielen 8-13 wurde jeweils mit Hilfe der ¹H-NMR-Spektroskopie bestimmt. Die gefundenen chemischen Verschiebungen waren wie folgt:

### Beispiel 8:

### (1-Methyl-5-piperidin-1-ylmethyl-1 H-pyrrol-2-yl)-piperidin-1-yl-essigsäureethylester Dihydrochlorid

δ (DMSO, 600 MHz): 1,19 (t, 3 H, J = 6,8 Hz, CH₃CH₂CO₂); 1,29 -1,42 (m, 2 H, N(CH₂CH₂)₂CH₂, N(CH₂CH₂)₂CH₂); 1,65 -1,97 (m, 10 H, N(CH₂CH₂)₂CH₂, N(CH₂CH₂)₂CH₂); 2,78 - 2,98 (m, 3 H, N(CH₂CH₂)₂CH₂, N(CH₂CH₂)₂CH₂); 3,02 - 3,10 (m, 1 H, N(CH₂CH₂)₂CH₂); 3,26 - 3,39 (m, 3 H, N(CH₂CH₂)_{Z}CH₂); 3,51 - 3,62 (m, 1 H, N(CH₂CH₂)₂CH₂); 3,84 (s, 3 H, NCH₃); 4,18 - 4,37 (m, 4 H, CH₂N(CH₂CH₂)₂CH₂, CH₃CH₂CO₂); 5,55 (d, 1 H, J = 6,8 Hz, CHCO₂CH₂CH₃); 6,35 - 6,40 (m, 1 H, N(CH₃)]-CCHCHC-[); 6,44 - 6,51 (m, 1 H, N(CH₃)]-CCHCHC-[); 10,50 (s, 1H, HCI); 11,83 (s, 1 H, HCI).

### Beispiel 9:

### (1-Methyl-5-piperidin-1-ylmethyl-1 H-pyrrol-2-yl)-morpholin-4-yl-essigsäureethylester Dihydrochlorid

δ (DMSO, 600 MHz) = 1,20 (t, 3 H, J = 6,8 Hz, C*H*₃CH₂CO₂); 1,30 -1,42 (m, 1 H, N(CH₂CH₂)₂C*H*₂); 1,66 -1,95 (m, 5 H, N(CH₂CH₂)₂C*H*₂, N(CH₂C*H*₂)₂CH₂); 2,77-3,15 (m, 4 H, N(C*H*₂CH₂)₂CH₂, 3,26 - 3,41 (m, 4 H, N(C*H*₂CH₂)₂O); 3,71 - 4,06 (m, 4 H, N(CH₂C*H*₂)₂O); 3,83 (s, 3 H, NC*H*₃); 4,16 - 4,37 (m, 4 H, C*H*₂N(CH₂CH₂)₂O, CH₃C*H*₂CO₂); 5,42 - 5,77 (br. s, 1 H, C*H*CO₂CH₂CH₃); 6,20 - 6,54 (m, 2 H, N(CH₃)]-CC*H*C*H*C-[); 10,79 (s, 1 H, HCl); 11,37 (s, 1 H, HCl).

### Beispiel 10:

### (5-Diethylaminomethyl-1-methyl-1 H-pyrrol-2-yl)-morpholin-4-yl-essigsäureethylester Dihydrochlorid

δ (DMSO, 600 MHz): 1,12 -1,43 (m, 9 H, CH₃CH₂CO₂, N(CH₂C*H*₃)₂); 2,81 - 4, 52 (m, 19 H, N(C*H*₂CH₂)₂O, N(CH₂CH₂)₂O, NC*H*₃, CH₃CH₂CO₂, N(C*H*₂CH₃)₂, C*H*₂N(CH₂CH₃)₂); 5,60 - 5,86 (br. s, 1 H, C*H*CO₂CH₂CH₃); 6,26 - 6,59 (m, 2 H, N(CH₃)]-CC*HCHC*-[); 10,89 (s, 1 H, HCl); 11,54 (s, 1 H, HCl).

### Beispiel 11:

### (5-Diethylaminomethyl-1-methyl-1 H-pyrrol-2-yl)-pyrrolidin-1-yl-essigsäureethylester Dihydrochlorid

δ (DMSO, 600 MHz): 1,18 (t, 3 H, *J* = 7,2 Hz, C*H*₃CH₂CO₂); 1,26 (t, 3 H, *J* = 7,2 Hz, N(CH₂C*H*₃)₂); 1,30 (t, 3 H, *J* = 7,2 Hz, N(CH₂C*H*₃)₂); 1,79 - 2,09 (m, 4 H, N(C*H*₂)₂(CH₂)₂); 2,95 - 3, 23 (m, 6 H, CH₃C*H*₂CO₂, N(C*H*₂CH₂)₂); 3,28 - 3,41 (m, 2 H, N(C*H*₂CH₂)₂); 3,86 (s, 3 H, NC*H*₃); 4,18 - 4,43 (m, 4 H, C*H*₂N(CH₂CH₃)₂, CH₃C*H*₂CO₂); 5,76 - 5,85 (m, 1 H, C*H*CO₂CH₂CH₃); 6,41 - 6,51 (m, 2 H, N(CH₃)]-CC*H*C*H*C-[); 10,83 (s, 1 H, HCl); 11,38 (s, 1 H, HCl).

### Beispiel 12:

### (1-Methyl-5-morpholin-4-ylmethyl-1 H-pyrrol-2-yl)-piperidin-1-yl-essigsäureethylester Dihydrochlorid

δ (DMSO, 600 MHz): 1,19 (t, 3 H, J = 7,2 Hz, C*H*₃C*H*₂CO₂); 1,29 -1,41 (m, N(CH₂CH₂)₂C*H*₂); 1,63 -1,97 (m, 5 H, N(CH₂CH₂)₂CH₂, N(CH₂CH₂)₂CH₂); 2,76 - 2,88 (m, 1 H, N(CH₂CH₂)₂CH₂); 3,04 - 3,21 (m, 3 H, N(CH₂CH₂)₂CH₂); 3,23 - 3,61 (m, 4 H, N(CH₂CH₂)₂0); 3,84 - 4,02 (m, 7 H, NCH₃, N(CH₂CH₂)₂O); 4,13 - 4,49 (m, 4 H, CH₃CH₂CO₂, C*H*₂N(CH₂CH₂)₂O); 5,58 (d, 1 H, J = 6,0 Hz, C*H*CO₂CH₂CH₃); 6,36 - 6,42 (m, 1 H, N(CH₃)]-CCHCHC-[); 6,49 - 6,54 (m, 1 H, N(CH₃)]-CC*H*C*H*C-[); 10,57 (s, 1H, HCl); 11,74 (s, 1H, HCl).

### Beispiel 13:

### (1-Methyl-5-morpholin-4-ylmethyl-1 H-pyrrol-2-yl)-pyrrolidin-1-yl-essigsäureethylester Dihydrochlorid

δ (DMSO, 600 MHz): 1,18 (t, 3 H, J = 7,2 Hz, C*H*₃CH₂CO₂); 1,78 - 2,13 (m, 4 H, N(CH₂C*H*₂)₂); 2,96 - 3,38 (m, 8 H, N(CH₂CH₂)₂, N(CH₂CH₂)₂0); 3,86 (s, 3 H, NCH₃); 3,90 - 4,00 (m, 4 H, N(CH₂CH₂)₂0); 4,17 - 4,29 (m, 2 H, CH₂N(CH₂CH₂)₂0); 4,30 - 4,46 (m, 2 H, CH₃CH₂CO₂); 5,80 (d, 1 H, J = 7,6 Hz, CHCO₂CH₂CH₃); 6,44 - 6,53 (m, 2 H, N(CH₃)]-CCHCHC-[); 11,37 (s, 1 H, HCl); 11,63 (s, 1 H, HCl).

### Pharmakologische Untersuchungen

### a) Affinität zum ORL-1-Rezeptor

Die Affinität der substituierten 2,5-Diaminomethyl-1 H-pyrrole zu dem ORL-1-Rezeptor wurde wie vorstehend ausgeführt bestimmt. Der Wert für eine ausgewählte Verbindung ist in der nachfolgenden Tabelle 5 wiedergegeben:

**Tabelle 5:**

| Verbindung gemäß Beispiel | ORL1-human Inhibierung [%] | ORL1-human Ki [µM] |
|---|---|---|
| 7 | 42 | 0,42 |

### b) µ-Affinität

Die Affinität der der substituierten 2,5-Diaminomethyl-1 H-pyrrole zum µ-Opioid-Rezeptor wurde wie vorstehend beschrieben bestimmt. Der Wert für einige ausgewählte Verbindungen ist in der nachfolgenden Tabelle 6 wiedergegeben:

**Tabelle 6:**

| Verbindung gemäß Beispiel | ORm-human Nal Inhibierung [%] | ORm-human Nal Ki [µM] |
|---|---|---|
| 2 | 25 | * |
| 5 | 52 | 0,86 |
| 6 | 21 | 0,5 |
| 7 | 50 | * |
| 8 | 69 | 0,22 |
| 10 | 84 | 0,07 |

| | | |
|---|---|---|
| * nicht bestimmt | | |

### c1) 5-HT-Uptake-Inhibierung

Die 5-HT-Uptake-Inhibierung der substituierten 2,5-Diaminomethyl-1 H-pyrrole wurde wie vorstehend beschrieben bestimmt. Die Werte für einige ausgewählte Verbindungen sind in der nachfolgenden Tabelle 7 wiedergegeben:

**Tabelle 7:**

| Verbindung gemäß Beispiel | Uptake 5-HT-Ratte Inhibierung [%] Conc. 10 |
|---|---|
| 1 | 77 |
| 2 | 76 |
| 3 | 30 |
| 4 | 57 |
| 5 | 39 |
| 6 | 53 |
| 7 | 44 |
| 9 | 26 |
| 10 | 28 |
| 13 | 28 |

### c2) Noradrenalin-Wiederaufnahmehemmung (Noradrenalin-Uptakehemmung)

Die Noradrenalin-Uptake-Inhibierung der substituierten 2,5-Diaminomethyl-1 H-pyrrole wie vorstehend beschrieben bestimmt. Die Werte für einige ausgewählte Verbindungen sind in der nachfolgenden Tabelle 8 wiedergegeben:

**Tabelle 8:**

| Verbindung gemäß Beispiel | Uptake Noradrenalin-Ratte Inhibierung [%], Conc. 10 |
|---|---|
| 1 | 63 |
| 2 | 65 |
| 5 | 91 |
| 6 | 42 |
| 7 | 52 |
| 8 | 33 |

## Patentansprüche

1. Arzneimittel enthaltend wenigstens ein substituiertes 2,5-Diaminomethyl-1 H-pyrrol der allgemeinen Formel I, 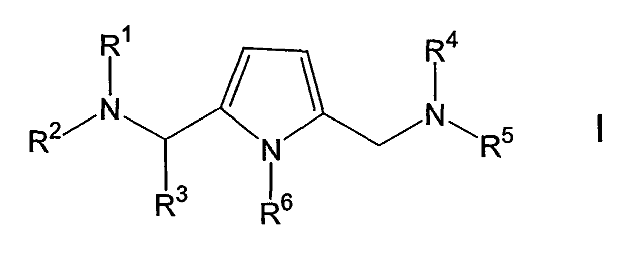 worin
R¹ für Wasserstoff, einen linearen oder verzweigten, gesättigten oder ungesättigten, ggf. wenigstens einfach substituierten aliphatischen Rest, einen ggf. über eine ggf. wenigstens einfach substituierte Alkylen-Gruppe gebundenen, gesättigten oder ungesättigten, ggf. wenigstens einfach substituierten, ggf. wenigstens ein Heteroatom als Ringglied aufweisenden cycloaliphatischen Rest, einen ggf. wenigstens einfach substituierten Aryl- oder Heteroaryl-Rest oder einen über eine ggf. substituierte Alkylen-Gruppe gebundenen, ggf. wenigstens einfach substituierten Aryl- oder Heteroarylrest steht,
R² für einen linearen oder verzweigten, gesättigten oder ungesättigten, ggf. wenigstens einfach substituierten aliphatischen Rest, einen ggf. über eine ggf. wenigstens einfach substituierte Alkylen-Gruppe gebundenen, gesättigten oder ungesättigten, ggf. wenigstens einfach substituierten, ggf. wenigstens ein Heteroatom als Ringglied aufweisenden cycloaliphatischen Rest, einen ggf. wenigstens einfach substituierten Aryl- oder Heteroaryl-Rest oder einen über eine ggf. substituierte Alkylen-Gruppe gebundenen, ggf. wenigstens einfach substituierten Aryl- oder Heteroarylrest steht,
oder
R¹ und R² gemeinsam mit dem sie verbindenden Stickstoffatom als Ringglied einen gesättigten oder ungesättigten, ggf. wenigstens ein weiteres Heteroatom als Ringglied aufweisenden, ggf. wenigstens einfach substituierten cycloaliphatischen Rest bilden,
R³ für einen ggf. wenigstens einfach substituierten Aryl- oder Heteroarylrest, eine Estergruppe oder eine Carboxygruppe steht,
R⁴ und R⁵, gleich oder verschieden, für Wasserstoff, einen linearen oder verzweigten, gesättigten oder ungesättigten, ggf. wenigstens einfach substituierten aliphatischen Rest, einen ggf. über eine ggf. wenigstens einfach substituierte Alkylen-Gruppe gebundenen, gesättigten oder ungesättigten, ggf. wenigstens einfach substituierten, ggf. wenigstens ein Heteroatom als Ringglied aufweisenden cycloaliphatischen Rest, einen ggf. wenigstens einfach substituierten Aryl- oder Heteroaryl-Rest oder einen über eine ggf. wenigstens einfach substituierte Alkylen-Gruppe gebundenden, ggf. wenigstens einfach substituierten Aryl- oder Heteroarylrest stehen,
oder
R⁴ und R⁵ gemeinsam mit dem sie verbindenden Stickstoffatom als Ringglied einen gesättigten oder ungesättigten, ggf. wenigstens ein weiteres Heteroatom als Ringglied aufweisenden, ggf. wenigstens einfach substituierten cycloaliphatischen Rest bilden, und
R⁶ für einen linearen oder verzweigten, gesättigten oder ungesättigten, ggf. wenigstens einfach substituierten aliphatischen Rest, einen ggf. über eine ggf. wenigstens einfach substituierte Alkylen-Gruppe gebundenen, gesättigten oder ungesättigten, ggf. wenigstens einfach substituierten, ggf. wenigstens ein Heteroatom als-Ringglied aufweisenden cycloaliphatischen Rest, einen ggf. wenigstens einfach substituierten Aryl- oder Heteroaryl-Rest oder einen über eine ggf. wenigstens einfach substituierte Alkylen-Gruppe gebundenden, ggf. wenigstens einfach substituierten Aryl- oder Heteroarylrest steht, gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

2. Arzneimittel gemäß Anspruch 1, **dadurch gekennzeichnet, daß**
R¹ für Wasserstoff, einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, fünf- oder sechsgliedrigen Aryl- oder Heteroarylrest oder einen über eine ggf. substituierte C₁₋₃-Alkylen-Gruppe gebundenen, ggf. wenigstens einfach substituierten, fünf- oder sechsgliedrigen Aryl- oder Heteroarylrest, vorzugsweise für Wasserstoff oder einen linearen oder verzweigten, gesättigten aliphatischen C₁₋₃-Rest, besonders bevorzugt für einen Methyl- oder Ethyl-Rest, steht.

3. Arzneimittel gemäß Anspruch 1 oder 2, **dadurch gekennzeichnet, daß**
R² für einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, fünf- oder sechsgliedrigen Aryl- oder Heteroarylrest oder einen über eine ggf. substituierte C₁₋₃-Alkylen-Gruppe gebundenen, ggf. wenigstens einfach substituierten, fünf- oder sechsgliedrigen Aryl- oder Heteroarylrest, vorzugsweise für Wasserstoff oder einen linearen oder verzweigten, gesättigten aliphatischen C₁₋₃-Rest, besonders bevorzugt für einen Methyl- oder Ethyl-Rest, steht.

4. Arzneimittel gemäß Anspruch 1, **dadurch gekennzeichnet, daß**
R¹ und R² gemeinsam mit dem sie verbindenden Stickstoffatom als Ringglied einen gesättigten oder ungesättigten, ggf. wenigstens einfach substituierten fünf- oder sechsgliedrigen cycloaliphatischen Rest bilden, der ggf. wenigstens ein weiteres Heteroatom ausgewählt aus der Gruppe bestehend aus N, O und S als Ringglied aufweist, vorzugsweise gemeinsam mit dem sie verbindenden Stickstoffatom als Ringglied einen gesättigten, ggf. Sauerstoff als weiteres Ringglied aufweisenden, fünf- oder sechsgliedrigen cycloaliphatischen Rest bilden, besonders bevorzugt gemeinsam für einen (CH₂)₄-, (CH₂)₅- oder (CH₂)₂-O-(CH₂)₂-Rest stehen, der mit dem sie verbindenden Stickstoffatom als Ringglied einen Heterocyclus bildet

5. Arzneimittel gemäß einem der Ansprüche 1 bis 4, **dadurch gekennzeichnet, daß**
R³ für einen unsubstituierten oder wenigstens einfach substituierten fünf- oder sechsgliedrigen Aryl- oder Heteroarylrest oder für eine Arylester, Heteroarylester oder Alkylester-Gruppe, vorzugsweise für einen ggf. wenigstens einfach substituierten Phenylrest oder eine Alkylester-Gruppe mit C₁₋₃, vorzugsweise mit C₁-C₂, im Alkylteil, steht.

6. Arzneimittel gemäß einem der Ansprüche 1 bis 5, **dadurch gekennzeichnet, daß**
R⁴ und R⁵, gleich oder verschieden, jeweils für Wasserstoff, einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, 5- oder 6-gliedrigen Aryl- oder Heteroarylrest oder einen über eine ggf. wenigstens einfach substituierte C₁₋₃₋Alkylen-Gruppe gebundenen, ggf. wenigstens einfach substituierten, 5- oder 6-gliedrigen Aryl- oder Heteroarylrest, vorzugsweise für Wasserstoff oder einen linearen oder verzweigten, gesättigten aliphatischen C₁₋₃-Rest, besonders bevorzugt für einen Methyl- oder Ethyl-Rest, stehen.

7. Arzneimittel gemäß einem der Ansprüche 1 bis 5, **dadurch gekennzeichnet, daß** R⁴ und R⁵ gemeinsam mit dem sie verbindenden Stickstoffatom als Ringglied einen gesättigten oder ungesättigten, ggf. wenigstens einfach substituierten fünf-, sechs- oder siebengliedrigen cycloaliphatischen Rest bilden, der ggf. wenigstens ein weiteres Heteroatom ausgewählt aus der Gruppe bestehend aus N, O und S als Ringglied aufweist, vorzugsweise gemeinsam mit dem sie verbindenden Stickstoffatom als Ringglied einen gesättigten, ggf. Sauerstoff als weiteres Ringglied aufweisenden, fünf- oder sechsgliedrigen cycloaliphatischen Rest bilden, besonders bevorzugt gemeinsam für einen (CH₂)₄-, (CH₂)₅- oder (CH₂)₂-O-(CH₂)₂-Rest stehen, der mit dem sie verbindenden Stickstoffatom als Ringglied einen Heterocyclus bildet

8. Arzneimittel gemäß einem der Ansprüche 1 bis 7, **dadurch gekennzeichnet, daß** der Rest R⁶ für einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden, cycloaliphatischen C₃₋₈-Rest, einen ggf. wenigstens einfach substituierten, 5- oder 6-gliedrigen Aryl- oder Heteroarylrest oder einen über eine ggf. wenigstens einfach substituierte C₁₋₃-Alkylen-Gruppe gebundenen, ggf. wenigstens einfach substituierten, 5- oder 6-gliedrigen Aryl- oder Heteroarylrest, vorzugsweise für einen linearen oder verzweigten, gesättigten aliphatischen C₁₋₃-Rest, besonders bevorzugt für eine Methylgruppe, steht.

9. Arzneimittel gemäß einem oder mehreren der Ansprüche 1 bis 8 enthaltend wenigstens ein 2,5-Diaminomethyl-1 H-pyrrol ausgewählt aus der Gruppe bestehend aus
[(2-Methoxyphenyl)-(1-methyl-5-piperidin-1-ylmethyl-1H-pyrrol-2-yl)-methyl]-dimethylamin,
[(2-Methoxyphenyl)-(1-methyl-5-pyrrolidin-1-ylmethyl-1H-pyrrol-2-yl)-methyl]dimethylamin,
Dimethyl-[(1-methyl-5-piperidin-1-ylmethyl-1H-pyrrol-2-yl)-o-tolyl-methyl]amin,
[(2-Bromphenyl)-(1-methyl-5-piperidin-1-ylmethyl-1H-pyrrol-2-yl)-methyl]-dimethylamin,
[(4-Bromphenyl)-(1-methyl-5-piperidin-1-ylmethyl-1H-pyrrol-2-yl)-methyl]-dimethylamin,
[(4-Fluorphenyl)-(1-methyl-5-morpholin-4-ylmethyl-1 H-pyrrol-2-yl)-methyl]dimethylamin,
[5-(Dimethylaminophenylmethyl)-1-methyl-1 H-pyrrol-2-ylmethyl]-diethylamin,
1-Methyl-5-piperidin-1-ylmethyl- 1H-pyrrol-2-yl)piperidin-1-yl-essigsäureethylester,
(1-Methyl-5-piperidin-1-ylmethyl-1 H-pyrrol-2-yl)morpholin-4-yl-essigsäureethylester,
(5-Diethylaminomethyl-1-methyl-1H-pyn-ol-2-yl)morpholin-4-yl-essigsäureethylester,
(5-Diethylaminomethyl-1 -methyl-1 H-pyrrol-2-yl)-pyrrolidin-1-yl-essigsäureethylester,
(1-Methyl-5-morpholin-4-ylmethyl-1 H-pyrrol-2-yl)-piperidin-1-yl-essigsäureethylester
und
(1-Methyl-5-morpholin-4-ylmethyl-1 H-pyrrol-2-yl)-pyrrolidin-1-yl-essigsäureethylester,
gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

10. Arzneimittel gemäß einem oder mehreren der Ansprüche 1 bis 9 zur Bekämpfung von Schmerzen.

11. Arzneimittel gemäß Anspruch 10 zur Bekämpfung von chronischen Schmerzen.

12. Arzneimittel gemäß Anspruch 10 zur Bekämpfung von akuten Schmerzen.

13. Arzneimittel gemäß Anspruch 10 zur Bekämpfung von neuropathischen Schmerzen.

14. Arzneimittel gemäß einem oder mehreren der Ansprüche 1 bis 9 zur Behandlung von Entzugserscheinungen.

15. Arzneimittel gemäß einem oder mehreren der Ansprüche 1 bis 9 zur Behandlung von Gedächtnis-Störungen.

16. Arzneimittel gemäß einem oder mehreren der Ansprüche 1 bis 9 zur Behandlung von neurodegenerativen Erkrankungen, vorzugsweise Morbus Parkinson, Morbus Huntington und/oder Morbus Alzheimer.

17. Arzneimittel gemäß einem oder mehreren der Ansprüche 1 bis 9 zur Behandlung von Epilepsie.

18. Arzneimittel gemäß einem oder mehreren der Ansprüche 1 bis 9 zur Diurese.

19. Arzneimittel gemäß einem oder mehreren der Ansprüche 1 bis 9 zur Behandlung cardiovaskulärer Störungen.

20. Arzneimittel gemäß einem oder mehreren der Ansprüche 1 bis 9 zur Behandlung von Wasserspeicher-Krankheiten.

21. Arzneimittel gemäß einem oder mehreren der Ansprüche 1 bis 9 zur Behandlung von intestinaler Motilität (Diarrhoe).

22. Arzneimittel gemäß einem oder mehreren der Ansprüche 1 bis 9 zur Unterdrückung des Miktionsreflexes.

23. Arzneimittel gemäß einem oder mehreren der Ansprüche 1 bis 9 zur Behandlung von Harninkontinenz.

24. Arzneimittel gemäß einem oder mehreren der Ansprüche 1 bis 9 zur Behandlung von Anorexie.

25. Arzneimittel gemäß einem oder mehreren der Ansprüche 1 bis 9 zur Behandlung von Pruritus.

26. Arzneimittel gemäß einem oder mehreren der Ansprüche 1 bis 9 zur Behandlung von Depressionen.

27. Arzneimittel gemäß einem oder mehreren der Ansprüche 1 bis 9 zur Anxiolyse.

28. Arzneimittel gemäß einem oder mehreren der Ansprüche 1 bis 9 zur Regulation des Elektrolyt-Haushaltes.

29. Arzneimittel gemäß einem oder mehreren der Ansprüche 1 bis 9 zur Beeinflussung des cardiovaskulären Systems, vorzugsweise zur Vasodilatation der Arterien.

30. Arzneimittel gemäß einem oder mehreren der Ansprüche 1 bis 9 zur Regulation, insbesondere Stimulation, der Nahrungsaufnahme.

31. Arzneimittel gemäß einem oder mehreren der Ansprüche 1 bis 9 zur Reduzierung des Suchtpotentials von Opioiden, insbesondere von Morphinen.

32. Arzneimittel gemäß einem oder mehreren der Ansprüche 1 bis 9 zur Modulation der Bewegungsaktivität.

33. Arzneimittel gemäß einem oder mehreren der Ansprüche 1 bis 9 zur Beeinflußung des cardiovaskulären Systems.

34. Arzneimittel gemäß einem oder mehreren der Ansprüche 1 bis 9 zur Behandlung von Tinnitus.

35. Arzneimittel gemäß einem oder mehreren der Ansprüche 1 bis 9 zur Behandlung von sexuellen Dysfunktionen, vorzugsweise erektiler Dysfunktion.

36. Arzneimittel gemäß einem oder mehreren der Ansprüche 1 bis 9 zur Behandlung von Atemwegserkrankungen.

37. Verwendung wenigstens eines substituierten 2,5-Diaminomethyl-1H-pyrrols gemäß den Ansprüchen 1-9 zur Herstellung eines Arzneimittels zur Bekämpfung von Schmerzen, vorzugsweise zur Bekämpfung von akuten Schmerzen und/oder chronischen Schmerzen und/oder neuropathischen Schmerzen.

38. Verwendung wenigstens eines substituierten 2,5-Diaminomethyl-1H-pyrrols gemäß den Ansprüchen 1-9 zur Herstellung eines Arzneimittels zur Behandlung von Entzugsersrheinungen. Gedächtnis-Störungen, neurodegenerativen Erkrankungen, vorzugsweise Morbus Parkinson, Morbus Huntington oder Morbus Alzheimer, Epilepsie, cardiovaskulärer Störungen, Wasserspeicher-Krankheiten, intestinaler Motilität (Diarrhoe).. Haminkontinenz, Anorexie. Tinitus. Pruritus, Depressionen, sexueller Dysfunktionen, vorzugsweise erektiler Dysfunktion. Atemwegserkrankungen, oder zur Anxiolyse, zur Diurese, zur Unterdrückung des Miktionsreflexes, zur Regulation, vorzugsweise Stimulation, der Nahrungsaufnahme, zur Reduzierung des Suchtpotentials von Opioiden, vorzugsweise von Morphin, zur Modulation der Bewegungsaktivität, oder zur Beeinflussung des cardiovaskulären Systems, vorzugsweise zur Vasodilatation der Arterien.

## Claims

1. A pharmaceutical preparation containing at least one substituted 2,5-diaminomethyl-1H-pyrrole of the general formula I, 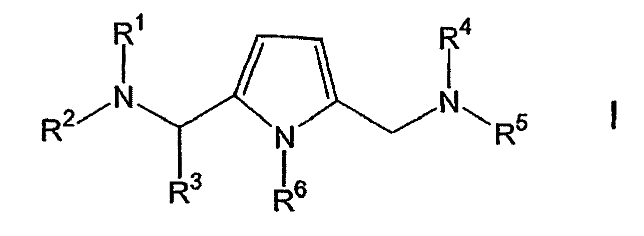 in which
R¹ denotes hydrogen, a linear or branched, saturated or unsaturated, optionally at least mono-substituted aliphatic residue, a saturated or unsaturated, optionally at least-mono-substituted, cycloaliphatic residue optionally comprising at least one heteroatom as a ring member and optionally attached via an optionally at least mono-substituted alkylene group, an optionally at least mono-substituted aryl or heteroaryl residue or an optionally at least mono-substituted aryl or heteroaryl residue attached via an optionally substituted alkylene group,
R² denotes a linear or branched, saturated or unsaturated, optionally at least mono-substituted aliphatic residue, a saturated or unsaturated, optionally at least mono-substituted, cycloaliphatic residue optionally comprising at least one heteroatom as a ring member and optionally attached via an optionally at least mono-substituted alkylene group, an optionally at least mono-substituted aryl or heteroaryl residue or an optionally at least mono-substituted aryl or heteroaryl residue attached via an optionally substituted alkylene group,
or
R¹ and R², together with the nitrogen atom joining them as a ring member, form a saturated or unsaturated, optionally at least mono-substituted cycloaliphatic residue optionally comprising at least one further heteroatom as a ring member,
R³ denotes an optionally at least mono-substituted aryl or heteroaryl residue, an ester group or a carboxy group,
R⁴ and R⁵, identical or different, denote hydrogen, a linear or branched, saturated or unsaturated, optionally at least mono-substituted aliphatic residue, a saturated or unsaturated, optionally at least mono-substituted, cycloaliphatic residue optionally comprising at least one heteroatom as a ring member and optionally attached via an optionally at least mono-substituted alkylene group, an optionally at least mono-substituted aryl or heteroaryl residue or an optionally at least mono-substituted aryl or heteroaryl residue attached via an optionally at least mono-substituted alkylene group,
or
R⁴ and R⁵, together with the nitrogen atom joining them as a ring member, form a saturated or unsaturated, optionally at least mono-substituted cycloaliphatic residue optionally comprising at least one further heteroatom as a ring member, and
R⁶ denotes a linear or branched, saturated or unsaturated, optionally at least mono-substituted aliphatic residue, a saturated or unsaturated, optionally at least mono-substituted, cycloaliphatic residue optionally comprising at least one heteroatom as a ring member and optionally attached via an optionally at least mono-substituted alkylene group, an optionally at least mono-substituted aryl or heteroaryl residue or an optionally at least mono-substituted aryl or heteroaryl residue attached via an optionally at least mono-substituted alkylene group,
optionally in the form of the pure stereoisomers thereof, in particular enantiomers or diastereomers, the racemates thereof or in the form of mixtures of the stereoisomers, in particular the enantiomers or diastereomers, in any desired mixing ratio, or in each case in the form of the acids or bases thereof or in the form of the salts thereof, in particular the physiologically acceptable salts, or in each case in the form of the solvates thereof, in particular the hydrates.

2. A pharmaceutical preparation according to claim 1, **characterised in that**
R¹ denotes hydrogen, a linear or branched, saturated or unsaturated, optionally at least mono-substituted aliphatic C₁₋₆ residue, a saturated or unsaturated, optionally at least monosubstituted cycloaliphatic C₃₋₈ residue optionally comprising at least one heteroatom as a ring member and optionally attached via an optionally at least mono-substituted C₁₋₃ alkylene group, an optionally at least mono-substituted, five- or six-membered aryl or heteroaryl residue or an optionally at least mono-substituted, five- or six-membered aryl or heteroaryl residue attached via an optionally substituted C₁₋₃ alkylene group, preferably hydrogen or a linear or branched, saturated aliphatic C₁₋₃ residue, particularly preferably a methyl or ethyl residue.

3. A pharmaceutical preparation according to claim 1 or claim 2, **characterised in that**
R² denotes a linear or branched, saturated or unsaturated, optionally at least mono-substituted aliphatic C₁₋₆ residue, a saturated or unsaturated, optionally at least monosubstituted cycloaliphatic C₃₋₈ residue optionally comprising at least one heteroatom as a ring member and optionally attached via an optionally at least mono-substituted C₁₋₃ alkylene group, an optionally at least mono-substituted, five- or six-membered aryl or heteroaryl residue or an optionally at least mono-substituted, five- or six-membered aryl or heteroaryl residue attached via an optionally substituted C₁₋₃ alkylene group, preferably hydrogen or a linear or branched, saturated aliphatic C₁₋₃ residue, particularly preferably a methyl or ethyl residue.

4. A pharmaceutical preparation according to claim 1, **characterised in that**
R¹ and R², together with the nitrogen atom joining them as a ring member, form a saturated or unsaturated, optionally at least mono-substituted, five- or six-membered cycloaliphatic residue, which optionally comprises at least one further heteroatom selected from the group consisting of N, O and S as a ring member, preferably, together with the nitrogen atom joining them as a ring member, form a saturated, five- or six-membered cycloaliphatic residue optionally comprising oxygen as a further ring member, particularly preferably together denote a (CH₂)₄, (CH₂)₅ or (CH₂)₂-O-(CH₂)₂ residue which, with the nitrogen atom joining them as a ring member, forms a heterocycle.

5. A pharmaceutical preparation according to any one of claims 1 to 4, **characterised in that**
R³ denotes an unsubstituted or at least mono-substituted, five- or six-membered aryl or heteroaryl residue or an aryl ester, heteroaryl ester or alkyl ester group, preferably an optionally at least mono-substituted phenyl residue or an alkyl ester group with C₁₋₃, preferably C₁-C₂, in the alkyl moiety.

6. A pharmaceutical preparation according to any one of claims 1 to 5, **characterised in that**
R⁴ and R⁵, identical or different, in each case denote hydrogen, a linear or branched, saturated or unsaturated, optionally at least mono-substituted aliphatic C₁₋₆ residue, a saturated or unsaturated, optionally at least monosubstituted cycloaliphatic C₃₋₈ residue optionally comprising at least one heteroatom as a ring member and optionally attached via an optionally at least mono-substituted C₁₋₃ alkylene group, an optionally at least mono-substituted, 5- or 6-membered aryl or heteroaryl residue or an optionally at least mono-substituted 5- or 6-membered aryl or heteroaryl residue attached via an optionally at least mono-substituted C₁₋₃ alkylene group, preferably hydrogen or a linear or branched, saturated aliphatic C₁₋₃ residue, particularly preferably a methyl or ethyl residue.

7. A pharmaceutical preparation according to any one of claims 1 to 5, **characterised in that** R⁴ and R⁵, together with the nitrogen atom joining them as a ring member, form a saturated or unsaturated, optionally at least mono-substituted, five-, six- or seven-membered cycloaliphatic residue, which optionally comprises at least one further heteroatom selected from the group consisting of N, O and S as a ring member, preferably, together with the nitrogen atom joining them as a ring member, form a saturated, five- or six-membered cycloaliphatic residue optionally comprising oxygen as a further ring member, particularly preferably together denote a (CH₂)₄, (CH₂)₅ or (CH₂)₂-O-(CH₂)₂ residue which, with the nitrogen atom joining them as a ring member, forms a heterocycle.

8. A pharmaceutical preparation according to any one of claims 1 to 7, **characterised in that** the residue R⁶ denotes a linear or branched, saturated or unsaturated, optionally at least mono-substituted aliphatic C₁₋₆ residue, a saturated or unsaturated, optionally at least monosubstituted cycloaliphatic C₃₋₈ residue optionally comprising at least one heteroatom as a ring member and optionally attached via an optionally at least mono-substituted C₁₋₃ alkylene group, an optionally at least mono-substituted, 5- or 6-membered aryl or heteroaryl residue or an optionally at least mono-substituted 5- or 6-membered aryl or heteroaryl residue attached via an optionally at least mono-substituted C₁₋₃ alkylene group, preferably a linear or branched, saturated aliphatic C₁₋₃ residue, particularly preferably a methyl group.

9. A pharmaceutical preparation according to one or more of claims 1 to 8 containing at least one 2,5-diaminomethyl-1H-pyrrole selected from the group consisting of
[(2-methoxyphenyl)-(1-methyl-5-piperidin-1-ylmethyl-1H-pyrrol-2-yl)-methyl]-dimethylamine,
[(2-methoxyphenyl)-(1-methyl-5-pyrrolidin-1-ylmethyl-1H-pyrrol-2-yl)-methyl]dimethylamine,
dimethyl-[(1-methyl-5-piperidin-1-ylmethyl-1H-pyrrol-2-yl)-o-tolyl-methyl]amine,
[(2-bromophenyl)-(1-methyl-5-piperidin-1-ylmethyl-1H-pyrrol-2-yl)-methyl]-dimethylamine,
[(4-bromophenyl)-(1-methyl-5-piperidin-1-ylmethyl-1H-pyrrol-2-yl)-methyl]-dimethylamine,
[(4-fluorophenyl)-(1-methyl-5-morpholin-4-ylmethyl-1H-pyrrol-2-yl)-methyl]-dimethylamine,
[5-(dimethylaminophenylmethyl)-1-methyl-1H-pyrrol-2-ylmethyl]-diethylamine,
1-methyl-5-piperidin-1-ylmethyl-1H-pyrrol-2-yl)piperidin-1-yl acetic acid ethyl ester,
(1-methyl-5-piperidin-1-ylmethyl-1H-pyrrol-2-yl)morpholin-4-yl acetic acid ethyl ester,
(5-diethylaminomethyl-1-methyl-1H-pyrrol-2-yl)morpholin-4-yl acetic acid ethyl ester,
(5-diethylaminomethyl-1-methyl-1H-pyrrol-2-yl)-pyrrolidin-1-yl acetic acid ethyl ester,
(1-methyl-5-morpholin-4-ylmethyl-1H-pyrrol-2-yl)-piperidin-1-yl acetic acid ethyl ester
and
(1-methyl-5-morpholin-4-ylmethyl-1H-pyrrol-2-yl)-pyrrolidin-1-yl acetic acid ethyl ester,
optionally in the form of the pure stereoisomers thereof, in particular enantiomers or diastereomers, the racemates thereof or in the form of mixtures of the stereoisomers, in particular the enantiomers or diastereomers, in any desired mixing ratio, or in each case in the form of the acids or bases thereof or in the form of the salts thereof, in particular the physiologically acceptable salts, or in the form of the solvates thereof, in particular the hydrates.

10. A pharmaceutical preparation according to one or more of claims 1 to 9 for combatting pain.

11. A pharmaceutical preparation according to claim 10 for combatting chronic pain.

12. A pharmaceutical preparation according to claim 10 for combatting acute pain.

13. A pharmaceutical preparation according to claim 10 for combatting neuropathic pain.

14. A pharmaceutical preparation according to one or more of claims 1 to 9 for the treatment of withdrawal symptoms.

15. A pharmaceutical preparation according to one or more of claims 1 to 9 for the treatment of memory disorders.

16. A pharmaceutical preparation according to one or more of claims 1 to 9 for the treatment of neurodegenerative diseases, preferably Parkinson's disease, Huntington's chorea and/or Alzheimer's disease.

17. A pharmaceutical preparation according to one or more of claims 1 to 9 for the treatment of epilepsy.

18. A pharmaceutical preparation according to one or more of claims 1 to 9 for diuresis.

19. A pharmaceutical preparation according to one or more of claims 1 to 9 for the treatment of cardiovascular disorders.

20. A pharmaceutical preparation according to one or more of claims 1 to 9 for the treatment of water retention disorders.

21. A pharmaceutical preparation according to one or more of claims 1 to 9 for the treatment of intestinal motility (diarrhoea).

22. A pharmaceutical preparation according to one or more of claims 1 to 9 for the suppression of the urinary reflex.

23. A pharmaceutical preparation according to one or more of claims 1 to 9 for the treatment of urinary incontinence.

24. A pharmaceutical preparation according to one or more of claims 1 to 9 for the treatment of anorexia.

25. A pharmaceutical preparation according to one or more of claims 1 to 9 for the treatment of pruritus.

26. A pharmaceutical preparation according to one or more of claims 1 to 9 for the treatment of depression.

27. A pharmaceutical preparation according to one or more of claims 1 to 9 for anxiolysis.

28. A pharmaceutical preparation according to one or more of claims 1 to 9 for regulating the electrolyte balance.

29. A pharmaceutical preparation according to one or more of claims 1 to 9 for influencing the cardiovascular system, preferably for vasodilating the arteries.

30. A pharmaceutical preparation according to one or more of claims 1 to 9 for regulating, in particular stimulating, food intake.

31. A pharmaceutical preparation according to one or more of claims 1 to 9 for reducing the addictive potential of opioids, in particular of morphines.

32. A pharmaceutical preparation according to one or more of claims 1 to 9 for modulating locomotor activity.

33. A pharmaceutical preparation according to one or more of claims 1 to 9 for influencing the cardiovascular system.

34. A pharmaceutical preparation according to one or more of claims 1 to 9 for the treatment of tinnitus.

35. A pharmaceutical preparation according to one or more of claims 1 to 9 for the treatment of sexual dysfunction, preferably erectile dysfunction.

36. A pharmaceutical preparation according to one or more of claims 1 to 9 for the treatment of respiratory diseases.

37. Use of at least one substituted 2,5-diaminomethyl-1H-pyrrole according to claims 1-9 for the production of a pharmaceutical preparation for combatting pain, preferably for combatting acute pain and/or chronic pain and/or neuropathic pain.

38. Use of at least one substituted 2,5-diaminomethyl-1H-pyrrole according to claims 1-9 for the production of a pharmaceutical preparation for the treatment of withdrawal symptoms, memory disorders, neurodegenerative diseases, preferably Parkinson's disease, Huntington's chorea or Alzheimer's disease, epilepsy, cardiovascular disorders, water retention conditions, intestinal motility (diarrhoea), urinary incontinence, anorexia, tinnitus, pruritus, depression, sexual dysfunction, preferably erectile dysfunction, respiratory diseases, or for anxiolysis, for diuresis, for suppression of the urinary reflex, for regulating, preferably stimulating, food intake, for reducing the addictive potential of opioids, preferably of morphine, for modulating locomotor activity, or for influencing the cardiovascular system, preferably for vasodilating the arteries.

## Revendications

1. Médicaments contenant au moins un 2,5-diaminométhyl-1H-pyrrole substitué de formule générale I 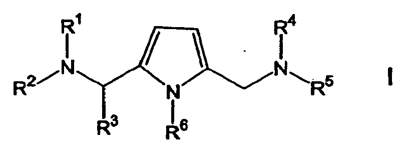 dans laquelle
R¹ est un atome d'hydrogène, un radical aliphatique à chaîne droite ou ramifiée, saturé ou insaturé, éventuellement au moins monoinsaturé, un radical cycloaliphatique, éventuellement au moins monosubstitué, éventuellement lié par l'intermédiaire d'un groupe alkylène éventuellement au moins monosubstitué, saturé ou insaturé, comportant éventuellement au moins un hétéroatome en tant que chaînon, un radical aryle ou hétéroaryle éventuellement au moins monosubstitué, ou encore un radical aryle ou hétéroaryle éventuellement au moins monosubstitué, lié par l'intermédiaire d'un groupe alkylène éventuellement substitué,
R² est un radical aliphatique à chaîne droite ou ramifiée, saturé ou insaturé, éventuellement au moins monosubstitué, un radical cycloaliphatique, éventuellement au moins monosubstitué, éventuellement lié par l'intermédiaire d'un groupe alkylène éventuellement au moins monosubstitué, saturé ou insaturé, comportant éventuellement au moins un hétéroatome en tant que chaînon, un radical aryle ou hétéroaryle éventuellement au moins monosubstitué, ou encore un radical aryle ou hétéroaryle, éventuellement au moins monosubstitué lié par l'intermédiaire d'un groupe alkylène éventuellement substitué,
ou
R¹ et R² forment, avec l'atome d'azote qui les relie en tant que chaînon, un radical cycloaliphatique éventuellement au moins monosubstitué, saturé ou insaturé, comportant éventuellement au moins un autre hétéroatome en tant que chaînon,
R³ est un radical aryle ou hétéroaryle éventuellement au moins monosubstitué, un groupe ester ou un groupe carboxy,
R⁴ et R⁵ sont identiques ou différents et représentent chacun un atome d'hydrogène, un radical aliphatique à chaîne droite ou ramifiée, saturé ou insaturé, éventuellement au moins monosubstitué, un radical cycloaliphatique éventuellement au moins monosubstitué, éventuellement lié par l'intermédiaire d'un groupe alkylène éventuellement au moins monosubstitué, saturé ou insaturé, comportant éventuellement au moins un hétéroatome en tant que chaînon, un radical aryle ou hétéroaryle éventuellement au moins monosubstitué, ou un radical aryle ou hétéroaryle éventuellement au moins monosubstitué, lié par l'intermédiaire d'un groupe alkylène éventuellement au moins monosubstitué,
ou
R⁴ et R⁵, avec l'atome d'azote qui les relie en tant que chaînon, forment un radical cycloaliphatique saturé ou insaturé, éventuellement au moins monosubstitué, comportant éventuellement au moins un autre hétéroatome en tant que chaînon,
R⁶ est un radical aliphatique à chaîne droite ou ramifiée, saturé ou insaturé, éventuellement au moins monosubstitué, un radical cycloaliphatique éventuellement au moins monosubstitué, éventuellement lié par l'intermédiaire d'un groupe alkylène éventuellement au moins monosubstitué, saturé ou insaturé, comportant éventuellement au moins un hétéroatome en tant que chaînon, un radical aryle ou hétéroaryle éventuellement au moins monosubstitué, ou encore un radical aryle ou hétéroaryle éventuellement au moins monosubstitué, lié par l'intermédiaire d'un groupe alkylène éventuellement au moins monosubstitué,
éventuellement sous forme de leurs stéréoisomères purs, notamment de leurs énantiomères ou diastéréoisomères, de leurs racémates, ou encore sous forme de mélanges des stéréoisomères, en particulier des énantiomères ou des diastéréoisomères, selon un rapport de mélange quelconque, ou encore, dans chaque cas, sous forme de leurs acides ou de leurs bases, ou sous forme de leurs sels, en particulier de leurs sels compatibles d'un point de vue physiologique, ou encore, dans chaque cas, sous forme de leurs produits de solvatation, notamment de leurs hydrates.

2. Médicaments selon la revendication 1, **caractérisés en ce que**
R¹ est un atome d'hydrogène, un radical aliphatique en C₁₋₆ à chaîne droite ou ramifiée, saturé ou insaturé, éventuellement au moins monosubstitué, un radical cycloaliphatique en C₃₋₈ éventuellement au moins monosubstitué, éventuellement lié par l'intermédiaire d'un groupe alkylène en C₁₋₃ éventuellement au moins monosubstitué, saturé ou insaturé, comportant éventuellement au moins un hétéroatome en tant que chaînon, un radical aryle ou hétéroaryle à 5 ou 6 chaînons, éventuellement au moins monosubstitué, ou encore un radical aryle ou hétéroaryle à 5 ou 6 chaînons éventuellement au moins monosubstitué, lié par l'intermédiaire d'un groupe alkylène en C₁₋₃ éventuellement substitué, de préférence un atome d'hydrogène, ou un radical aliphatique en C₁₋₃ à chaîne droite ou ramifiée, et saturé, d'une manière particulièrement préférée un groupe méthyle ou éthyle.

3. Médicaments selon la revendication 1 ou 2, **caractérisés en ce que**
R² est un radical aliphatique en C₁₋₆ à chaîne droite ou ramifiée, saturé ou insaturé, éventuellement au moins monosubstitué, un radical cycloaliphatique en C₃₋₈ éventuellement au moins monosubstitué, éventuellement lié par l'intermédiaire d'un groupe alkylène en C₁₋₃ éventuellement au moins monosubstitué, saturé ou insaturé, comportant éventuellement au moins un hétéroatome en tant que chaînon, un radical aryle ou hétéroaryle à 5 ou 6 chaînons, éventuellement au moins monosubstitué, ou encore un radical aryle ou hétéroaryle à 5 ou 6 chaînons, éventuellement au moins monosubstitué, lié par l'intermédiaire d'un groupe alkylène en C₁₋₃ éventuellement substitué, de préférence un atome d'hydrogène ou un radical aliphatique en C₁₋₃ à chaîne droite ou ramifiée, et saturé, d'une manière particulièrement préférée un groupe méthyle ou éthyle.

4. Médicaments selon la revendication 1, **caractérisés en ce que**
R¹ et R ² avec l'atome d'azote qui les relie en tant que chaînon, forment un radical cycloaliphatique à 5 ou 6 chaînons, saturé ou insaturé, éventuellement au moins monosubstitué, radical qui comprend éventuellement au moins un autre hétéroatome choisi dans l'ensemble consistant en N, O et S en tant que chaînon, et de préférence, avec l'atome d'azote qui les relie en tant que chaînon, forment un radical cycloaliphatique à 5 ou 6 chaînons, saturé, comportant éventuellement de l'oxygène en tant qu'autre chaînon, et d'une manière particulièrement préférée représentent ensemble un radical (CH₂)₄-, (CH₂)₅- ou (CH₂)₂-O-(CH₂)₂-, qui avec l'atome d'azote qui les relie en tant que chaînon forme un radical hétérocyclique.

5. Médicaments selon l'une des revendications 1 à 4, **caractérisés en ce que**
R³ représente un radical aryle ou hétéroaryle à 5 ou 6 chaînons, non substitué ou au moins monosubstitué, ou encore un groupe ester arylique, ester hétéroarylique ou ester alkylique, de préférence un radical phényle éventuellement au moins monosubstitué, ou un groupe ester alkylique ayant dans le fragment alkyle 1 à 3, et de préférence 1-2 atomes de carbone.

6. Médicaments selon l'une des revendications 1 à 5, **caractérisés en ce que**
R⁴ et R⁵ sont identiques ou différents et représentent chacun un atome d'hydrogène, un radical aliphatique en C₁₋₆ à chaîne droite ou ramifiée, saturé ou insaturé, éventuellement au moins monosubstitué, un radical cycloaliphatique en C₃₋₈ éventuellement au moins monosubstitué, éventuellement lié par l'intermédiaire d'un groupe alkylène en C₁₋₃ éventuellement au moins monosubstitué, saturé ou insaturé, comportant éventuellement au moins un hétéroatome en tant que chaînon, un radical aryle ou hétéroaryle à 5 ou 6 chaînons, éventuellement au moins monosubstitué, ou encore un radical aryle ou hétéroaryle à 5 ou 6 chaînons, éventuellement au moins monosubstitué, lié par l'intermédiaire d'un groupe alkylène en C₁₋₃ éventuellement monosubstitué, de préférence un atome d'hydrogène ou un radical aliphatique en C₁₋₃ à chaîne droite ou ramifiée, saturé, d'une manière particulièrement préférée un groupe méthyle ou éthyle.

7. Médicaments selon l'une des revendications 1 à 5, **caractérisés en ce que** R⁴ et R⁵ forment avec l'atome d'azote qui les relie en tant que chaînon un radical cycloaliphatique à 5, 6 ou 7 chaînons, saturé ou insaturé, éventuellement au moins monosubstitué, radical qui comprend éventuellement au moins un autre hétéroatome choisi dans l'ensemble consistant en N, O et S en tant que chaînon, de préférence forment avec l'atome d'azote qui les relie en tant que chaînon un radical cycloaliphatique à 5 ou 6 chaînons, saturé, comportant éventuellement de l'oxygène en tant qu'autre chaînon, et d'une manière particulièrement préférée représentent ensemble un radical (CH₂)₄-, (CH₂)₅- ou (CH₂)₂-O-(CH₂)₂-, qui avec l'atome d'azote qui les relie en tant que chaînon forme un radical hétérocyclique.

8. Médicaments selon l'une des revendications 1 à 7, **caractérisés en ce que** le radical R⁶ est un radical aliphatique en C₁₋₆ à chaîne droite ou ramifiée, saturé ou insaturé, éventuellement au moins monosubstitué, un radical cycloaliphatique en C₃₋₈ éventuellement au moins monosubstitué, éventuellement lié par l'intermédiaire d'un groupe alkylène en C₁₋₃ éventuellement au moins monosubstitué, saturé ou insaturé, comportant éventuellement au moins un hétéroatome en tant que chaînon, un radical aryle ou hétéroaryle à 5 ou 6 chaînons, éventuellement au moins monosubstitué, ou encore un radical aryle ou hétéroaryle à 5 ou 6 chaînons éventuellement au moins monosubstitué, lié par l'intermédiaire d'un groupe alkylène en C₁₋₃ éventuellement au moins monosubstitué, de préférence un radical aliphatique en C₁₋₃ à chaîne droite ou ramifiée, et saturé, d'une manière particulièrement préférée un groupe méthyle.

9. Médicaments selon l'une ou plusieurs des revendications 1 à 8, contenant au moins un 2,5-diaminométhyl-1H-pyrrole choisi dans l'ensemble consistant en les composés suivants :
[(2-méthoxyphényl)-(1-méthyl-5-pipéridine-1-ylméthyl-1H-pyrrole-2-yl)-méthyl]diméthylamine,
[(2-méthoxyphényl)-(1-méthyl-5-pyrrolidine-1-ylméthyl-1H-pyrrole-2-yl)-méthyl]diméthylamine,
diméthyl-[(1-méthyl-5-pipéridine-1-ylméthyl-1H-pyrrole-2-yl)-o-tolyl-méthyl]amine,
[(2-bromophényl)-(1-méthyl-5-pipédirine-1-ylméthyl-1H-pyrrole-2-yl)-méthyl]diméthylamine,
[(4-bromophényl)-(1-méthyl-5-pipéridine-1-ylméthyl-1H-pyrrole-2-yl)-méthyl]diméthylamine,
[(4-fluorophényl)-(1-méthyl-5-morpholine-4-ylméthyl-1H-pyrrole-2-yl)-méthyl]diméthylamine,
[5-(diméthylaminophénylméthyl)-1-méthyl-1H-pyrrole-2-ylméthyl]diéthylamine,
ester éthylique de l'acide 1-méthyl-5-pipéridine-1-ylméthyl-1H-pyrrole-2-yl)pipéridine-1-yl-acétique,
ester méthylique de l'acide (1-méthyl-5-pipéridine-1-ylméthyl-1H-pyrrole-2-yl)morpholine-4-yl-acétique,
ester éthylique de l'acide (5-diéthylaminométhyl-1-méthyl-1H-pyrrole-2-yl)morpholine-4-yl-acétique,
ester éthylique de l'acide (5-diéthylaminométhyl-1-méthyl-1H-pyrrole-2-yl)pyrrolidine-1-yl-acétique,
ester éthylique de l'acide (1-méthyl-5-morpholine-4-ylméthyl-1H-pyrrole-2-yl)pipéridine-1-yl-acétique,
et
ester éthylique de l'acide (1-méthyl-5-morpholine-4-ylméthyl-1H-pyrrole-2-yl)pyrrolidine-1-yl-acétique,
éventuellement sous forme de leurs stéréoisomères purs, notamment de leurs énantiomères ou diastéréoisomères, de leurs racémates, ou encore sous forme de mélanges des stéréoisomères, en particulier des énantiomères ou des diastéréoisomères, selon un rapport de mélange quelconque, ou encore, dans chaque cas, sous forme de leurs acides ou de leurs bases, ou sous forme de leurs sels, en particulier de leurs sels compatibles d'un point de vue physiologique, ou encore, dans chaque cas, sous forme de leurs produits de solvatation, notamment de leurs hydrates.

10. Médicaments selon l'une ou plusieurs des revendications 1 à 9 pour lutter contre les douleurs.

11. Médicaments selon la revendication 10, pour lutter contre les douleurs chroniques.

12. Médicaments selon la revendication 10, pour lutter contre les douleurs aiguës.

13. Médicaments selon la revendication 10, pour lutter contre les douleurs neuropathiques.

14. Médicaments selon l'une ou plusieurs des revendications 1 à 9 pour le traitement des phénomènes de sevrage.

15. Médicaments selon l'une ou plusieurs des revendications 1 à 9 pour le traitement des troubles de la mémoire.

16. Médicaments selon l'une ou plusieurs des revendications 1 à 9 pour le traitement des maladies neurodégénératives, de préférence la maladie de Parkinson, la maladie de Huntington et/ou la maladie d'Alzheimer.

17. Médicaments selon l'une ou plusieurs des revendications 1 à 9 pour le traitement de l'épilepsie.

18. Médicaments selon l'une ou plusieurs des revendications 1 à 9 pour la diurèse.

19. Médicaments selon l'une ou plusieurs des revendications 1 à 9 pour le traitement des troubles cardiovasculaires.

20. Médicaments selon l'une ou plusieurs des revendications 1 à 9 pour le traitement des maladies liées aux ballons d'eau.

21. Médicaments selon l'une ou plusieurs des revendications 1 à 9 pour le traitement de la motilité intestinale (diarrhée).

22. Médicaments selon l'une ou plusieurs des revendications 1 à 9 pour réprimer le réflexe de miction.

23. Médicaments selon l'une ou plusieurs des revendications 1 à 9 pour le traitement de l'incontinence urinaire.

24. Médicaments selon l'une ou plusieurs des revendications 1 à 9 pour le traitement de l'anorexie.

25. Médicaments selon l'une ou plusieurs des revendications 1 à 9 pour le traitement du prurit.

26. Médicaments selon l'une ou plusieurs des revendications 1 à 9 pour le traitement des dépressions.

27. Médicaments selon l'une ou plusieurs des revendications 1 à 9 pour l'anxiolyse.

28. Médicaments selon l'une ou plusieurs des revendications 1 à 9 pour la régulation du bilan électrolytique.

29. Médicaments selon l'une ou plusieurs des revendications 1 à 9 pour influer sur le système cardiovasculaire, de préférence pour la vasodilatation des artères.

30. Médicaments selon l'une ou plusieurs des revendications 1 à 9 pour la régulation, en particulier la stimulation, de l'ingestion des aliments.

31. Médicaments selon l'une ou plusieurs des revendications 1 à 9 pour réduire le potentiel d'addiction d'opioïdes, notamment de morphines.

32. Médicaments selon l'une ou plusieurs des revendications 1 à 9 pour la modulation de l'activité motrice.

33. Médicaments selon l'une ou plusieurs des revendications 1 à 9 pour influer sur le système cardiovasculaire.

34. Médicaments selon l'une ou plusieurs des revendications 1 à 9 pour le traitement de l'acouphène.

35. Médicaments selon l'une ou plusieurs des revendications 1 à 9 pour le traitement des dysfonctionnements sexuels, de préférence du dysfonctionnement de l'érection.

36. Médicaments selon l'une ou plusieurs des revendications 1 à 9 pour le traitement des maladies des voies respiratoires.

37. Utilisation d'au moins un 2,5-diaminométhyl-1H-pyrrole selon l'une des revendications 1 à 9, pour préparer un médicament destiné à lutter contre les douleurs, de préférence à lutter contre les douleurs aiguës et/ou les douleurs chroniques et/ou les douleurs neuropathiques.

38. Utilisation d'au moins un 2,5-diaminométhyl-1H-pyrrole substitué selon les revendications 1 à 9 pour préparer un médicament destiné au traitement des phénomènes de sevrage, des troubles de la mémoire, des maladies neurodégénératives, de préférence la maladie de Parkinson, la maladie de Huntington ou la maladie d'Alzheimer, de l'épilepsie, des troubles cardiovasculaires, des maladies dues aux ballons d'eau, de la motilité intestinale (diarrhée), de l'incontinence urinaire, de l'anorexie, de l'acouphène, du prurit, des dépressions, des dysfonctionnements sexuels, de préférence du dysfonctionnement de l'érection, des maladies des voies respiratoires, ou pour l'anxiolyse, pour la diurèse, pour réprimer le réflexe de miction, pour la régulation, de préférence la stimulation, de l'ingestion d'aliments, pour la réduction du potentiel d'addiction d'opioïdes, de préférence de la morphine, pour la modulation de l'activité motrice, ou pour influer sur le système cardiovasculaire, de préférence pour la vasodilatation des artères.
